# EUROPEAN PATENT APPLICATION

(11) **EP 3 647 421 A1**
(43) Date of publication of application: **06.05.2020**
(21) Application number: 19207288.2
(22) Date of filing: 05.11.2019
(51) Int. Cl.: C12N 15/11, C12N 15/63, C12N 15/85, C12N 15/64, A61K 31/713

(54) **USING MINIVECTORS TO TREAT OVARIAN CANCER**

(30) Priority: 05.11.2018 US 201816180046
(71) Applicant: Baylor College of Medicine, Houston, TX 77030 (US); Twister Biotech, Inc., Houston TX 77225 (US)
(72) Inventor: ZECHIEDRICH, E. Lynn, Houston, TX 77225 (US); MATZUK, Martin M., Houston, TX 77030 (US); YEN, Laising, Houston, TX 77030 (US); YU, Zhifeng, Houston, TX 77030 (US); AREVALO-SOLIZ, Lirio Milenka, Houston, TX 77030 (US); CATANESE, Jr, Daniel James, Houston, TX 77030 (US); FOGG, Jonathan Marcus, Houston, TX 77030 (US); COKER, Christopher Elbert, Houston, TX 77030 (US)
(74) Representative: Barker Brettell LLP

(57) **Abstract**

MiniVectors and compositions containing MiniVectors that target ovarian cancer genes selected from FOXM1, AKT, CENPA, PLK1, CDC20, BIRC5, AURKB, CCNB1, CDKN3, BCAM-AKT2, CDKN2D-WDFY2, SLC25A6, CIP2A, CD133, ALDH1A1, CD44, SALL4, and/or PRDM16, alone or in any combination, are provided, along with uses in the treatment of ovarian cancer.

## Description

### PRIOR RELATED APPLICATIONS

This application claims priority to U.S. Serial No. 16/180,046, filed November 5, 2018, incorporated by reference in its entirety for all purposes.

### FEDERALLY SPONSORED RESEARCH STATEMENT

This invention was made with government support under grants R01GM115501, R56AI054830, R01AI054830 and R01CA060651 awarded by the National Institutes of Health. The government has certain rights in the invention.

### FIELD OF THE DISCLOSURE

The disclosure generally relates to methods of treating ovarian cancer using gene therapy and methods and combinations of methods to deliver gene therapy. It also relates to methods of making DNA MiniVectors and compositions comprising MiniVectors useful in treating ovarian and similar cancers having similar P53 and/or FOXM1 effects.

### BACKGROUND OF THE DISCLOSURE

Ovarian cancer refers to any cancerous growth that occurs in the ovary. According to the United States National Cancer Institute, ovarian cancer is the 8th most common cancer among women in the United States (excluding non-melanoma skin cancers). However, it is the 5th most common cause of cancer deaths in women. Each year, more than 22,000 women in the U.S. are diagnosed with ovarian cancer and around 14,000 will die. Tragically, the overall 5-year survival rate is only 46 percent in most developed countries (and survival rate is lower for more advanced stages). However, according to the National Cancer Institute, if diagnosis is made early, before the tumor has spread, the 5-year survival rate is 94 percent.

In addition to the long used chemical and radiological treatments for ovarian cancer, gene therapy is now of clinical interest in treating ovarian cancers, albeit still in its infancy. At least one clinical trial is studying insertion of the p53 gene into a person's cancer cells, which hopefully will improve the body's ability to fight cancer or make the cancer cells more sensitive to treatment. Another group has studied the use of carbonyl reductase 1 (CBR1) overexpression in a mouse model, in which DNA was delivered to ovarian cancer cells via a polyamidoamine (PAMAM) dendrimer and initial results were promising.

One of the most important objectives in gene therapy is the development of highly safe and efficient vector systems for gene transfer to eukaryotic cells. Initially, viral-based vector systems were used, most commonly retroviruses or adenoviruses, to deliver the desired gene. Other viruses used as vectors include adeno-associated viruses, lentiviruses, pox viruses, alphaviruses, and herpes viruses. The main advantage of virus-based vectors is that viruses have evolved to physically deliver a genetic payload into cells and this can be readily exploited. The efficiency of delivery into cells is therefore generally higher than non-viral delivery methods, e.g., plasmid DNAs.

Viral-based vectors can have disadvantages, however. Viruses can usually infect more than one type of cell and can infect healthy cells as well as diseased cells. Another danger is that the new gene might be inserted in the wrong location in the genome, possibly causing cancer or other problems. This has already occurred in clinical trials for X-linked severe combined immunodeficiency (X-SCID) patients. In addition, there is a small chance that viral DNA could unintentionally be introduced into the patient's reproductive cells, thus producing changes that may be passed on to children. Another concern is the possibility that transferred genes could be overexpressed, producing so much of the added gene product as to be harmful. Moreover, the viral vector could cause an immune reaction or could be transmitted from the patient to other individuals or even into the environment. Use of viruses is also burdened with concerns of subsequent virus mutation and reactivation. Perhaps most important, most viral vectors can often only be delivered once because of developed immunity; subsequent deliveries produce a strong immune response.

Plasmids could potentially be used instead of viral-based vectors. Plasmids are far less efficient at entering cells than viruses, but have utility because they are straightforward to generate and isolate. In fact, clinical trials using intramuscular injection of "naked" DNA plasmid have occurred with some success. Unfortunately, transfection of plasmids as well as expression from plasmids has been low in comparison to viral vectors-too low to affect disease in many cases.

Numerous studies have shown that the bacterial backbone in plasmids may elicit immune responses as well as cause reduction of transgene expression. Furthermore, the introduction of antibiotic resistance genes, often encoded on plasmids for propagation, is not allowed by some government regulatory agencies. Because of these issues, smaller DNA vectors, such as minicircles and MiniVectors, were developed. These non-viral DNA vectors are small (typically ≦5 kilobase pairs (kb) circular plasmid derivatives that are almost completely devoid of bacterial sequences including the genes for selection (often antibiotic resistance genes) and origins of DNA replication). They have been used as transgene carriers for the genetic modification of mammalian cells, with the advantage that, since they contain no bacterial DNA sequences, they are less likely to suffer from the well documented silencing of transgene expression that often occurs when the transgene is carried on a plasmid containing long bacterial sequences and are also less likely to elicit an immune response. Several studies have demonstrated that minicircles and MiniVectors are safe-they are episomal vectors that enhance transgene expression extent and duration *in vivo* and *in vitro* relative to plasmids.

The use of small vectors less than 1,000 bp is highly promising, but vectors of this small length were initially difficult to produce and purify in significant quantity. Site-specific recombination is inhibited when the recombination sites are closely spaced, and intermolecular recombination between sites on two separate plasmids becomes more favorable than bending such a short sequence on the same plasmid, leading to multimeric products (Fogg 2006). An alternative approach commonly used by experimentalists is the circularization of linear DNA molecules via ligation to form minicircles. However, yields are very low and intermolecular ligation contaminants are prevalent when the linear DNA molecules short enough to generate minicircles are used.

US7622252 overcame the problem of MiniVector yield and purity by transforming the plasmid into a cell suitable for site-specific recombination to occur, under conditions such that topoisomerase IV decatenation activity is inhibited, thereby producing catenated DNA circles wherein at least one of the circles in each catenane is a supercoiled DNA minicircle of less than about 1 kb in size.

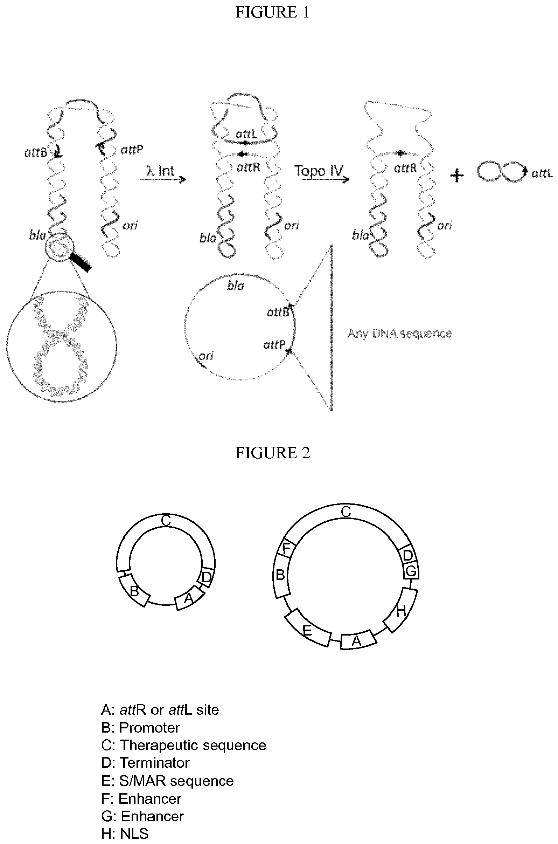

MiniVectors are minimized, non-viral DNA vectors similar to minicircles but with some important differences. Like minicircles, MiniVectors are synthesized from a parent plasmid via site-specific recombination. Encoding only the genetic payload and short integration sequences, MiniVectors can be engineered as small as ∼250-350 base pairs (bp) and generated in high yields (in comparison, the smallest reported minicircle length is 650 bp although the yields of minicircles of that length are unreported). As before, unwanted bacterial sequences are on a discarded miniplasmid. The recombination and purification system used to make MiniVectors is highly optimized, resulting in as much as 100-fold less plasmid contamination than is recommended by health regulatory agencies (0.015%). MiniVector preparation usually follows the basic procedure shown in **FIG. 1****.**

The different DNA species in the MiniVector purification process are typically engineered to be of sufficiently different lengths to be readily separated by size-exclusion chromatography (gel-filtration). This step is a unique and major advantage of the MiniVector system and enables the recovery of a highly pure preparation of MiniVector. By contrast, a minicircle, although similarly made initially, cannot be made as small as a MiniVector and is typically less pure, carrying along up to 10% of plasmid and other circle contaminants in the final product, which is a yield well above the 1.5% allowed by some health regulatory agencies.

What is needed in the art are better methods of conducting gene delivery, especially delivery of sequences specific for treating ovarian cancers. We are now using the materials and methods described in US7622252 to develop and test a variety of sequences for this use.

### SUMMARY OF THE DISCLOSURE

This application focuses on the treatment of ovarian cancer and improving the survival rate of patients by creating MiniVectors to specifically target key ovarian cancer targets and pathways. Below are some of the ovarian cancer targets for which we have data from cell culture and/or animal models testing novel therapeutics.

The MiniVectors are designed to target ovarian cancer inhibiting sequences, such as FOXM1, CENPA, PLK1, CDC20, BIRC5, AURKB, CCNB1, CDKN3, BCAM-AKT2, CDKN2D-WDFY2, SLC25A6, CIP2A, CD133, ALDH1A1, CD44, SALL4, and PRDM16, to name just a few.

The MiniVectors can contain a single ovarian cancer inhibitory gene, or it can contain more than one such sequence, although with increasing size, efficiency advantages start to be lost. Thus, in some embodiments, MiniVectors are used that are encoding single or multiple DNA sequences against one or multiple targets or multiple MiniVectors encoding single or multiple DNA sequences against one or multiple targets delivered in combination to reduce the expression of one or more targets simultaneously. In other embodiments, MiniVectors can be combined with other gene delivery vectors and/or other therapeutic agents.

While minicircles, plasmid, viruses, micelles, cationic lipids, and the like could be used herein for delivery of ovarian cancer inhibitory gene(s) or RNA sequences, MiniVectors are preferred, being easier to manufacture in purity and quantity and thus safer for human use. MiniVectors are small, circular, supercoiled DNA constructs ideal for RNA interference (e.g., shRNA knockdown), RNA activation, long term gene and RNA expression, cell labeling, and molecular studies of DNA structure and binding. In contrast to plasmids, MiniVectors are stripped down to the very essence of what one hopes to deliver in therapeutics: just the DNA sequence of interest.

MiniVectors offer one or more of the following advantages over other gene delivery vehicles. 1) MiniVector transfection efficiency is equal to siRNA and better than plasmid in several cell types tested. MiniVector DNA transfects every cell type we have tried, including: aortic smooth muscle cells, suspension lymphoma cells, and other difficult to transfect cell types. 2) MiniVector knockdown efficiency lasts longer than siRNA or plasmid because siRNA, shRNA, or miRNA are all rapidly degraded and thus require constant replenishment. Unlike therapeutically delivered RNAs, which typically rapidly degrade, MiniVectors are long-lasting. Unlike plasmids, which are silenced, MiniVectors are not silenced. 3) Smaller therapeutic MiniVectors survive exposure to human serum for at least three times longer than a typical larger sized therapeutic plasmid (there is a strong length dependence on digestion). Plasmids are typically too big to penetrate cells, so they require potentially toxic delivery methods. Finally, longer plasmids are highly susceptible to shear forces from nebulization. Resulting degraded linear DNA can trigger DNA repair and/or activate apoptosis. 4) MiniVectors withstand nebulization, making them an ideal delivery vector to lungs via aerosol. 5) MiniVectors successfully transfect T-cells, stem cells, and cancer cells.

A significant difference between MiniVectors and minicircles lies in the method of their purification. Minicircle purification relies upon (never 100% efficient) cleavage of the parent vector, leading to its degradation inside the bacterial cell, and fails to separate remaining uncleaved parent or the other recombination product from minicircle. Indeed, a recent review (Hardee et al. (2017) provides the following comparison:

| **Type of DNA vector** | **Advantages** | **Disadvantages** |
|---|---|---|
| Minicircle | Vectors have been designed that are appropriate for mammalian mitochondrial gene therapy | Some plasmid and other DNA contaminants can remain in the final product |
| MiniVector | Smallest circular DNA vector | Not well-known in the field |
| | Most supercoiled DNA vector | |
| | Greatest purity | |
| | Naked MiniVector <1200 bp resists nebulization shear forces | |

MiniVector is a double-stranded, supercoiled circular DNA typically lacking a bacterial origin of replication or an antibiotic selection gene, and having a length of about 250 bp up to about 5 kb. It is usually obtained by site-specific recombination of a parent plasmid to eliminate plasmid sequences outside of the recombination sites, but the sizes of the various components are designed to facilitate separation, and the separation is not *in vivo* restriction enzyme based by definition herein. Purity levels of MiniVectors are typically much higher than a minicircle preparation and there is usually, by gel electrophoresis analysis, no detectible contamination from catenanes, the other circular recombination product, or the parent plasmid. MiniVectors of very short lengths do sometimes become dimerized and sometimes trimerized (or higher multimers). These multimers do not constitute "contaminants" and they still contain only the therapeutic sequence but are merely double (or triple, etc.) the desired therapy. Slightly longer MiniVectors decrease the likelihood of multimers forming (Fogg et al. 2006). Furthermore, if a short MiniVector is desired, an extra gel filtration step typically separates higher multimers from single unit-sized MiniVector, if needed.

**FIG. 2** schematizes the modularity of MiniVectors. On the left is shown the simplest embodiment of a MiniVector consisting of (A) the hybrid DNA recombination sequences, *att*L or *att*R*,* that are products of the site-specific recombination, (B) a mammalian promoter, (C) the therapeutic DNA sequence to be expressed, and (D) a transcriptional terminator.

The MiniVector contains, for example, DNA encoding merely the transgene expression cassette (including promoter and a sequence of interest, wherein the nucleic acid sequence may be, for example, a gene, or a segment of a gene, a sequence encoding an interfering RNA (*e.g*., shRNA, lhRNA, miRNA, shRNA-embedded miRNA, lncRNA, piRNA), or a template for *e.g*., homology-directed repair, alteration, or replacement of the targeted DNA sequence). Importantly, the MiniVector is almost completely devoid of bacterial-originated sequences.

MiniVectors are designed to contain limited or no homology to the human genome. They are also typically shorter in length than plasmids. Therefore, the integration is at least as low as the 5 x 10⁻⁶ rate of plasmid integration and likely is lower. Designed to be delivered locally, any non-target would have to have MiniVector in the non-target cells/tissue to cause an off-target effect. In that way, then, MiniVectors should not have off-target effects. In contrast, viruses are designed to integrate into the genome, and therefore there is a major risk of off-target integration.

As used herein, "shape" encompasses the basic geometric shapes, such as star, rod, disc, and the like, as well as including features such as aspect ratio, local surface roughness, features in all three-dimensions, varied surface curvatures, the potential for creative and diverse biomimicry, numbers of surface appendages, extreme geometries, etc. "Shape" is best assessed by electron microscopy.

As used herein, "a defined geometric shape" means that the MiniVector has a particular geometric shape that is either transient or non-transient, *e.g*., is formed in or retained in solution *in vivo,* such as *e.g.,* a rod, a star, a hexagon, a cube or rhomboid, or a tetrahedron, and other specific shape. It expressly excludes linear or nicked DNAs that freely change shape in solution or ordinary supercoiled DNAs lacking a non-transient shape imposed thereon by design. Furthermore, the shape is a function of the DNA sequence, and is not only externally imposed thereon, *e.g.,* by histones, capsid proteins, ligands, or micelles, and the like.

As used herein, when we say that ∼50% of said MiniVectors have a specific shape, we mean that when visualized we see that more than half of the vectors have the same shape, although that shape appears differently when viewed from different angles.

As used herein, "non-transient" means that the shape is retained for a time long enough to be measured in solution. "Transient" shapes may also be useful such that cells may specifically take up such shapes when they transiently appear. These sequence-engineered shapes contrast from the various forms that a circular or linear DNA without engineered shape may take in solution, such as random linear DNA shapes or circles (nicked or supercoiled).

As used herein a "rod" is a generally cylindrical shape that is elongated and has an aspect ratio (AR) of > 2.4. A "microrod" is a rod that is at least 1 micron long in the long axis. Shape has been shown to influence cellular localization and uptake of synthetic nanoparticles *(e.g.,* gold nanorods, sugar structures, *etc.).* Particles with diameters on the order of microns (interestingly about the same size as a platelet) preferentially displace to the cell free layer (CFL) in the presence of red blood cells (RBCs), while smaller particles do not experience this enhanced localization. Although not yet directly tested with DNA, we anticipate that shape and size will similarly affect the cellular localization and uptake of DNA nanoparticles generated by incorporation of sequence-directed bends into the MiniVector DNA. A "nanorod" by contrast is of length in the long axis < 1 micron. Nanorods may accumulate significantly in the spleen.

As used herein, a "star" shape has a plurality of generally evenly sized and distributed projections, *e.g*., six armed stars have been shown to be preferentially delivered to pulmonary tissue when delivered intravenously.

As used herein, the term "RNA interference," or "RNAi," refers to the process whereby sequence-specific, post-transcriptional gene silencing is initiated by an RNA that is homologous in sequence to the silenced gene. RNAi, which occurs in a wide variety of living organisms and their cells, from plants to humans, has also been referred to as post-transcriptional gene silencing and co-suppression in different biological systems. The sequence-specific degradation of mRNA observed in RNAi is mediated by small (or short) interfering RNAs (siRNAs).

As used herein, the term "interfering RNA" means an RNA molecule capable of decreasing the expression of a gene having a nucleotide sequence at least a portion of which is substantially the same as that of the interfering RNA. As known in the art, interfering RNAs can be "small interfering RNAs," or siRNAs, composed of two complementary single-stranded RNAs that form an intermolecular duplex. Interfering RNAs can also be "long hairpin RNAs," or lhRNAs, which are shRNA-like molecules with longer intramolecular duplexes and contain more than one siRNA sequence within the duplex region.

As used herein, the term "gene silencing" refers to a reduction in the expression product of a target gene. Silencing may be complete, in that no final gene product is detectable, or partial, in that a substantial reduction in the amount of gene product occurs.

As used herein, "shRNA" is short hairpin RNA or small hairpin RNA, and "lhRNA" is long hairpin RNA, both of which can be used to silence target gene expression via RNAi.

As used herein, "miRNA" is microRNA-a small non-coding RNA molecule (containing about 22 nucleotides) found in plants, animals, and some viruses, that functions in RNA silencing and post-transcriptional regulation of gene expression. Alternative to a contiguous duplex shRNA is an shRNA sequence embedded in a microRNA stemloop (e.g., MiRE), which may be used because it can be processed more efficiently in mammalian cells leading to more robust knockdown of the expression of the target gene. The more efficient processing of the microRNA stemloop relies on both Drosha and Dicer, whereas the contiguous duplex shRNA relies only on Dicer to cut the guide RNA that will be inserted into the RNA-induced silencing complex.

As used herein, "lncRNA" are long non-coding RNAs. These lncRNAs are a large and diverse class of transcribed RNA molecules with a length of more than 200 nucleotides that do not encode proteins (or lack >100 amino acid open reading frame). lncRNAs are thought to encompass nearly 30,000 different transcripts in humans, hence lncRNA transcripts account for the major part of the non-coding transcriptome. lncRNA discovery is still at a preliminary stage. There are many specialized lncRNA databases, which are organized and centralized through RNAcentral (rnacentral.org). lncRNAs can be transcribed as whole or partial natural antisense transcripts to coding genes, or located between genes or within introns. Some lncRNAs originate from pseudogenes. lncRNAs may be classified into different subtypes (Antisense, Intergenic, Overlapping, Intronic, Bidirectional, and Processed) according to the position and direction of transcription in relation to other genes.

Piwi-interacting RNA or "piRNA" is the largest class of small non-coding RNA molecules expressed in animal cells. piRNAs form RNA-protein complexes through interactions with PIWI family proteins. These piRNA complexes have been linked to both epigenetic and post-transcriptional gene silencing of retrotransposons and other genetic elements in germ line cells, particularly those in spermatogenesis. They are distinct from miRNA in size (26-31 nt rather than 21-24 nt), lack of sequence conservation, and increased complexity.

The term "treating" includes both therapeutic treatment and prophylactic treatment (reducing the likelihood of disease development). The term means decrease, suppress, attenuate, diminish, arrest, or stabilize the development or progression of a disease *(e.g.,* a disease or disorder delineated herein), lessen the severity of the disease, or improve the symptoms associated with the disease.

As described herein, MiniVector for use in gene therapy is present in an effective amount. As used herein, the term "effective amount" refers to an amount which, when administered in a proper dosing regimen, is sufficient to treat (therapeutically or prophylactically) the target disorder or symptoms of the target disorder. For example, an effective amount is sufficient to reduce or ameliorate the severity, duration, or progression of the disorder being treated, prevent the advancement of the disorder being treated, cause the regression of the disorder being treated, or enhance or improve the prophylactic or therapeutic effect(s) of another therapy.

By "reducing" the expression of a target protein, we mean a reduction of at least 10%, as the body's own immune response may thereby be sufficient to target and kill the cancer cells, particularly in a combination therapy combined with an immune-boosting treatment, such as CpG motifs, cytokines (chemokines, interferons, interleukins, lymphokines, and tumor necrosis factors). Preferably the reduction is at least 20%, 30% or 40%, but typically a complete knockout is not required, and indeed, can contribute to unwanted side effects.

"Nanoparticles" are understood to comprise particles in any dimension that are less than 1,000 nanometers, more preferably less than 500 nanometers, and most preferably less than 300 nanometers. The nanoparticle can be a viral vector, a component of a viral vector (*e.g*., a capsid), a non-viral vector (*e.g.,* a plasmid or RNA or MiniVector), a cell, a fullerene and its variants, a small molecule, a peptide, metal and oxides thereof, etc.

The use of the word "a" or "an" when used in conjunction with the term "comprising" in the claims or the specification means either one or more than one, unless the context dictates otherwise. The term "about" means the stated value plus or minus the margin of error of measurement or plus or minus 25% if no method of measurement is indicated. The use of the term "or" in the claims is used to mean "and/or" unless explicitly indicated to refer to alternatives only or if the alternatives are mutually exclusive.

The terms "comprise," "have," "include," and "contain" (and their variants) are open-ended linking verbs and allow the addition of other elements when used in a claim. The phrase "consisting of' is closed, and excludes all additional elements. The phrase "consisting essentially of' excludes additional material elements but allows the inclusions of non-material elements that do not substantially change the nature of the invention, such as instructions for use, buffers, and the like.

The following abbreviations are used herein. Description includes UniProt accession numbers, when appropriate (NA, not applicable).

| | | |
|---|---|---|
| AKT2 | RAC-beta serine/threonine-protein kinase (gene AKT2) | P31751 |
| ALDH1A1 | Retinal dehydrogenase 1 | P00352 |
| AURKB | Aurora kinase B | Q96GD4 |
| BCAM | Basal Cell Adhesion Molecule | P50895 |
| BIRC5 | Baculoviral IAP repeat-containing protein 5 | O15392 |
| CCNB1 | G2/mitotic-specific cyclin-B1 | P14635 |
| CD133 | Prominin-1 | O43490 |
| CD44 | CD44 antigen, Receptor for hyaluronic acid (HA) | P16070 |
| CDC20 | Cell division cycle protein 20 homolog | Q12834 |
| CDKN2D | Cyclin-dependent kinase 4 inhibitor D | P55273 |
| CDKN3 | Cyclin-dependent kinase inhibitor 3 | Q16667 |
| CENPA | Histone H3-like centromeric protein A | P49450 |
| CIP2A | CIP2A (gene KIAA1524) | Q8TCG1 |
| FOXM1 | Forkhead box protein M1 | Q08050 |
| MAR | Matrix attachment region | NA |
| NLS | Nuclear localization signal | NA |
| PLK1 | Serine/threonine-protein kinase PLK1 | P53350 |
| PRDM16 | PR domain zinc finger protein 16 | Q9HAZ2 |
| S/MAR | Scaffold/matrix attachment region | NA |
| SALL4 | Sal-like protein 4 | Q9UJQ4 |
| SLC25A6 | ADP/ATP translocase 3 (gene SLC25A6) | P12236 |
| WDFY2 | WD repeat and FYVE domain-containing protein 2 | Q96P53 |

### BRIEF DESCRIPTION OF THE DRAWINGS

**FIG. 1****.** Generation of MiniVector DNA by λ-integrase-mediated site-specific recombination. Parent plasmid containing the sequence to be delivered flanked by *att*B and *att*P, the target sites for recombination. The parent plasmid is propagated in the special *E. coli* bacterial host strain, LZ54 or LZ31, harboring λ-integrase (Int) under the control of the temperature sensitive *c*I857 repressor. When the cells have reached a suitable density, expression of Int is switched on by a temperature switch. Recombination results in a catenated product containing the MiniVector. The products are decatenated, either by endonuclease cleavage of the large circle deletion product *ex vivo,* or by topoisomerase IV-mediated unlinking subsequent to the removal of topoisomerase inhibitor following the cell harvest. The deletion product containing the undesired bacterial sequences is removed, yielding pure, supercoiled MiniVector product. If desired, the MiniVector can encode *att*R and the deletion product can contain *att*L by switching the positions of *att*B and *att*P in the parent plasmid. B1a = beta lactamase.
**FIG. 2****.** Modular design of MiniVectors. On the left is shown the minimal therapeutic unit, consisting only of A) *att*L or *att*R site (these sites are the products of recombination by integrase), B) a promoter, C) the therapeutic sequence (*e.g*., shRNA encoding sequence), and D) a transcriptional terminator. Potential sequences for A, B and C are listed in Tables 1-3. The intervening regions can include any other sequence and can range in length from none to several thousand base pairs. On the right is shown a modified version containing additional modules that may be added to provide long-term persistence and expression, improve transfection, and/or facilitate nuclear localization. Any combination of these additional modules may be added to the essential modules. E) S/MAR sequence, if incorporated into the MiniVector, will be placed upstream of the transcriptional unit to utilize the dynamic negative supercoiling generated by transcription or elsewhere on the molecule. F, G) Enhancer sequences may be positioned in a number of locations, depending on the identity of the enhancer. H) Nuclear localization sequences, if incorporated, will be placed downstream of the transcriptional unit.

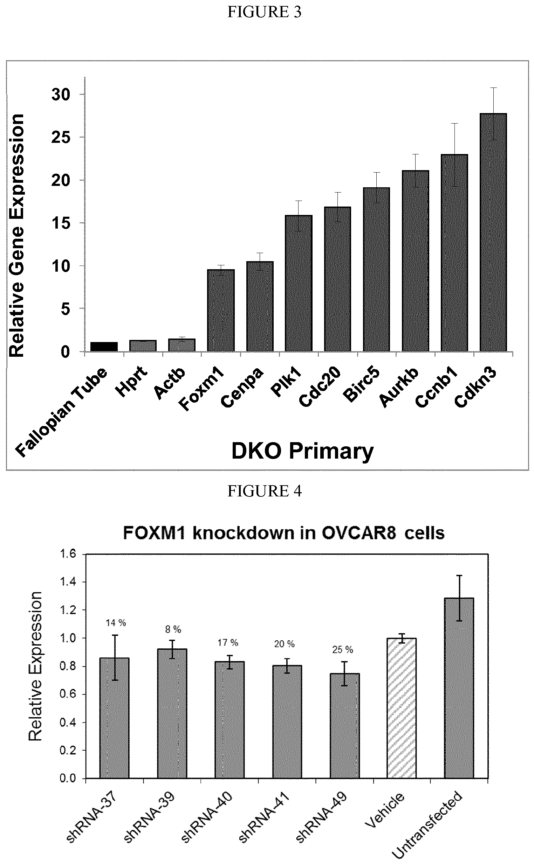

**FIG. 3****.** FOXM1 and downstream gene targets are upregulated in PTEN/DICER1 double cKO tumors. This cancer mouse model spontaneously grows high grade serous ovarian tumors up-regulating the same genes as in humans. Among these genes is FOXM1.
**FIG. 4****.** Selecting an effective shRNA against FOXM1 in an ovarian cancer cell line. The experiment shown was at 72 h post-transfection with 4 µg plasmid DNA encoding the various shRNAs. Gene knockdown was quantified by qPCR. This experiment was repeated twice with three replicates in each experiment. The actin gene was used as an endogenous control. Percentage knockdown is shown relative to the vehicle only control.

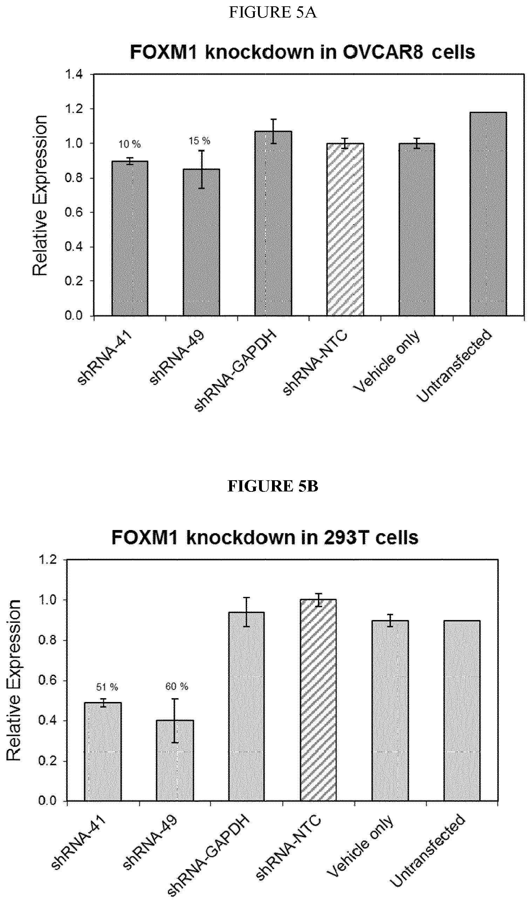

**FIG. 5A-B**. Effect of shRNA on FOXM1 expression using plasmid delivery in (A) Ovarian cancer cells or (B) non-cancer 293T cells. The experiments shown were from 48 h post-transfection with 2 µg DNA. The actin gene was used as an endogenous control. Percentage knockdown is shown from qPCR data compared to the shRNA scrambled control (NTC).

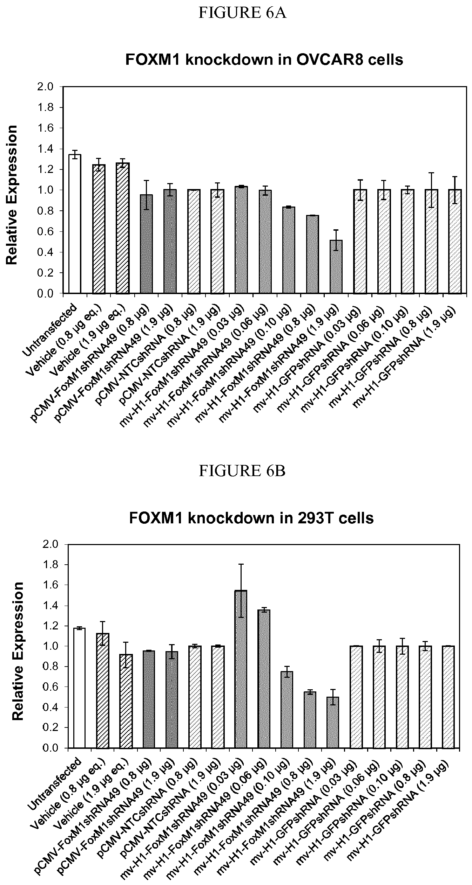

**FIG. 6A****:** FoxM1 Knockdown via MiniVectors in OVCAR8. **FIG. 6B****:** FoxM1 knockdown via MiniVectors in 293T cells.

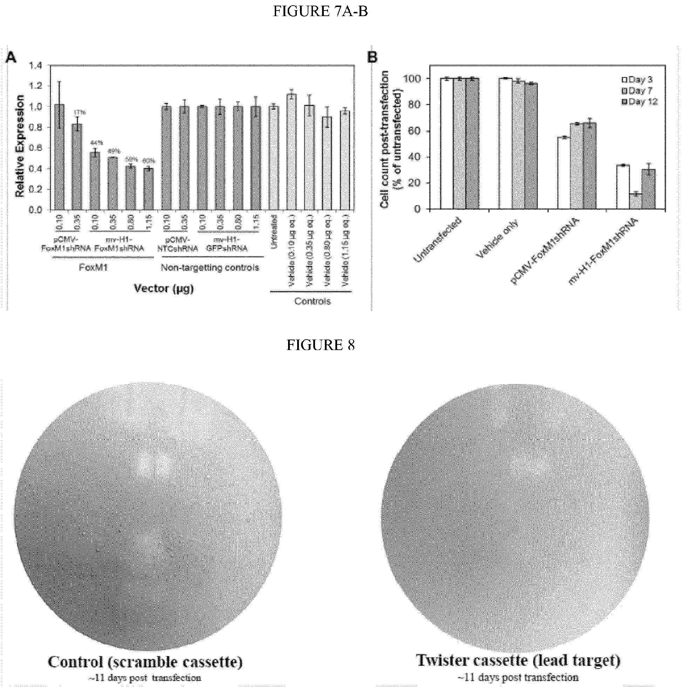

**FIG. 7A-B**. Comparison of knockdown of FOXM1 by plasmid (pCMV-FoxMlshRNA) or MiniVector (mv-FoxMlshRNA) encoding shRNA against FOXM1 in OVCAR8 cells **(A)** and effect on OVCAR8 cell viability **(B).** In panel A, plasmid (from Dharmacon, Inc.) or MiniVector encoding shRNA against FOXM1 were delivered via Lipofectamine 3000 to OVCAR8 cells; knockdown was assessed three days post-transfection. Plasmid encoding a "non-targeting control" (pCMV-NTCshRNA; also from Dharmacon, Inc.) or MiniVector encoding shRNA against GFP (mv-H1-GFPshRNA) were used as off-target controls. Gene expression was assessed by qRT-PCR. In panel B, OVCAR8 cell growth post-transfection was plotted as % of untransfected cells. Bars are the mean; error bars depict standard deviation from an experiment conducted in triplicate. The experiment was repeated two more times (each time in triplicate) with the same results. Statistical significance was determined using a student's t-test. *p* < 0.001 for all cases when error bars did not overlap.
**FIG 8****.** Minivector encoding shRNA against FOXM1 shows improved cell killing of OVCAR8 cells by microscopy. In the experiment described in **FIG. 7B**, MiniVector (labeled Twister cassette) shows very few OVCAR8 cells in culture on day 11 post-transfection as compared to the control sample.

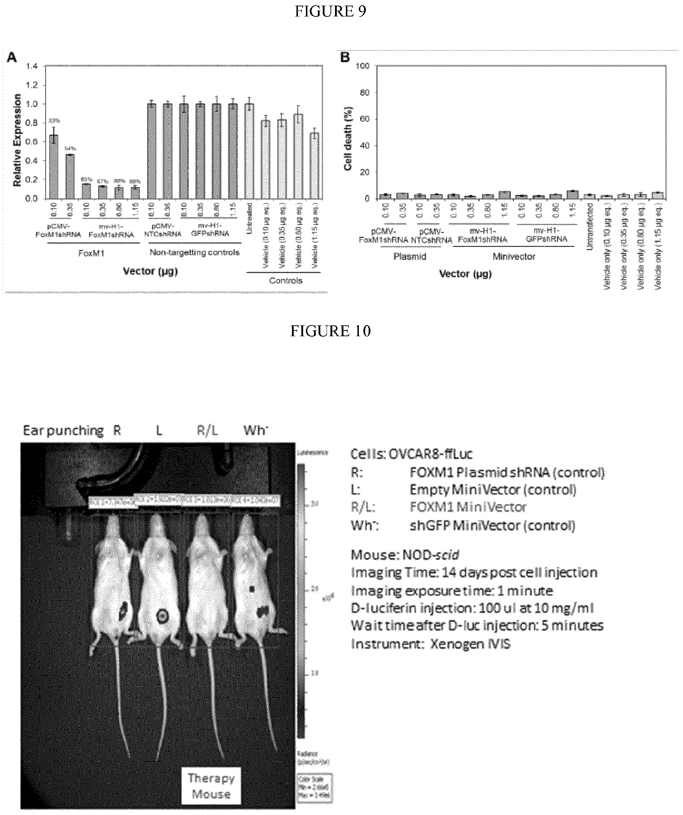

**FIG. 9A-B** Comparison of knockdown of FOXM1 by plasmid (pCMV-FOXM1shRNA) or MiniVector (mv-FoxM1shRNA) encoding shRNA against FOXM1 (A) and its effect on cell viability **(B)** of 293T cells. Plasmid or MiniVector encoding FOXM1 shRNA were delivered via Lipofectamine 3000 to 293T cells; knockdown was assessed three days post-transfection. The same controls as **FIG. 7** were used for off-target effects. The live/dead dye, NucBlue, was added, and the percentage of dead cells were quantified by flow cytometry. Bars are the mean; error bars depict standard deviation from the mean from an experiment conducted in triplicate. The experiment was repeated twice with the same results.
**FIG. 10****.** shRNA against FOXM1 were tested for efficacy in a high grade serous carcinoma (HGSC) xenograft mouse.

### DETAILED DESCRIPTION

The disclosure provides novel MiniVectors used to target and treat ovarian cancers. The invention includes any one or more of the following embodiment(s), in any combination(s) thereof:

| |
|---|
| A MiniVector, said MiniVector being a double-stranded, supercoiled, circular DNA encoding an ovarian cancer inhibitory sequence (OCi) that can be expressed in a eukaryotic cell. Said MiniVector typically lacks a bacterial origin of replication and lacks antibiotic resistance genes, but may if desired for some applications. Preferably, the MiniVector without the OCi is < 600 bp. |
| The invention also includes ovarian cancer inhibitory (OCi) RNA for a target gene selected from shRNA, miRNA, IncRNA, piRNA, RNAi, or antisense RNA. The OCi RNAs and DNAs encoding same are preferably for a target gene selected from FOXM1, CENPA, PLK1, CDC20, BIRC5, AURKB, CCNB1, CDKN3, BCAM-AKT2, CDKN2D-WDFY2, SLC25A6, CIP2A, CD133, ALDH1A1, CD44, SALL4, AKT1, AKT2, AKT3, and/or PRDM16 alone or in any combination. These OCi RNAs can be delivered by any method, but one preferred method is by delivery of a MiniVector encoding same. The OCi DNA could also be on a plasmid, virus, or minicircle, or other delivery means. |
| Any MiniVector, or composition thereof described herein, wherein said MiniVector is at least 95% pure. It could even be 97% pure, or 99.5% pure, 99.9% pure or higher (purity assessed with respect to contaminating parent plasmid DNA and recombination DNA side products). |
| Any MiniVector, or composition thereof described herein, wherein said MiniVector is at least 95% or 97% or 99% pure, is separated from parent plasmid and recombination side-products on the basis of size, and does not depend upon the use of a restriction cleavage *in vivo.* |
| Any MiniVector, or composition thereof described herein, wherein said MiniVector is at least 95%, 97%, 99% or 99.5% pure and does not contain detectable parent plasmid or recombination side-products. |
| A MiniVector, or composition thereof, wherein said OCi encodes an inhibitory RNA for a target gene selected from shRNA, miRNA, IncRNA, piRNA, RNAi, and/or antisense RNA alone or in any combination. |
| A MiniVector, or composition thereof, wherein said OCi encodes an inhibitory RNA for a target gene selected from FOXM1, CENPA, PLK1, CDC20, BIRC5, AURKB, CCNB1, CDKN3, BCAM-AKT2, CDKN2D-WDFY2, SLC25A6, CIP2A, CD133, ALDH1A1, CD44, SALL4, and/or PRDM16, alone or in any combination, and wherein expression of said target gene(s) is reduced by at least 10% by said inhibitory RNA when said MiniVector is introduced into mammalian cells. |
| A MiniVector, or composition thereof, wherein said ovarian cancer inhibitory sequence is an apoptosis gene selected from p53, p16, p21, p27, E2F genes, FHIT, PTEN, and/or CASPASE alone or in any combination, and said apoptosis gene is expressed when said MiniVector is introduced into eukaryotic cells. |
| A MiniVector, or composition thereof, comprising a promoter operably connected to said OCi operably connected to a terminator. |
| A MiniVector, or composition thereof, comprising a promoter connected to said OCi operably connected to a terminator, and additionally comprising an enhancer sequence and/or a nuclear localization signal (NLS). |
| A MiniVector, or composition thereof, wherein said MiniVector is expressible in a human cell and said OCi is for a human target gene. |
| A MiniVector, or composition thereof, that is made by: engineering a parent plasmid DNA molecule comprising site-specific recombination sites on either side of said OCi; transforming said parent plasmid into a cell suitable for site-specific recombination to occur, under conditions such that topoisomerase IV decatenation activity is inhibited, thereby producing a plurality of catenated DNA circles, wherein at least one of the circles in each catenane is a supercoiled DNA MiniVector of typically less than about 5 kb in length; decatenating the catenated site-specific recombination products, thereby releasing the supercoiled DNA MiniVector from the catenanes; and isolating the supercoiled DNA MiniVector. |
| Any MiniVector, or composition thereof, described herein, wherein said MiniVectors are 250 bp to 5,000 bp in total length. |
| Any MiniVector, or composition thereof, described herein, wherein said MiniVectors are <500 bp or <400 bp or <350 bp or <300 bp or <250 bp in length, excluding said OCi. |
| A composition comprising a MiniVector in a pharmaceutically acceptable excipient, said MiniVector being a double-stranded, supercoiled, nicked, or relaxed circular DNA encoding an ovarian cancer inhibitory sequence (OCi) and typically lacking a bacterial origin of replication and lacking an antibiotic resistance gene, wherein said circular DNA is at least 95% free of parent plasmid DNA or recombination side-products, wherein said OCi is expressible in human cells and thereby inhibits the expression of a human target gene selected from FOXM1, CENPA, PLK1, CDC20, BIRC5, AURKB, CCNB1, CDKN3, BCAM-AKT2, CDKN2D-WDFY2, SLC25A6, CIP2A, CD133, ALDH1A1, CD44, SALL4, and/or PRDM16, alone or in any combination. |
| Any MiniVector, or composition thereof, described herein, wherein said ovarian cancer inhibitory sequence is codon optimized to modify (decrease or increase) expression in humans or expression in specific cell types or specific cancer cell types. |
| Any MiniVector, or composition thereof described herein, wherein said MiniVector is CpG-free, CpG maximized, or CpG minimized. |
| Any MiniVector, or composition thereof described herein, that is supercoiled. Preferably, the DNA is at least 85% supercoiled, 90%, 95% or 99%. |
| A MiniVector, or composition thereof, which is relaxed or nicked. |
| A MiniVector, or composition thereof, said MiniVector being a double-stranded, supercoiled, circular DNA encoding an ovarian cancer inhibitory sequence (OCi) that can be expressed in a eukaryotic cell, wherein said OCi encodes an inhibitory RNA for a target gene selected from FOXM1, CENPA, PLK1, CDC20, BIRC5, AURKB, CCNB1, CDKN3, BCAM-AKT2, CDKN2D-WDFY2, SLC25A6, CIP2A, CD133, ALDH1A1, CD44, SALL4, and/or PRDM16 alone or in any combination, wherein said MiniVector typically lacks a bacterial origin of replication or antibiotic resistance gene, wherein said MiniVector is separated from a parent plasmid and recombination side-products on the basis of size, and does not use a restriction cleavage *in vivo,* and does not contain detectable parent plasmid or recombination side-products as measured by gel electrophoresis stain. |
| A MiniVector, or composition thereof, said MiniVector being a double-stranded, supercoiled, circular DNA encoding an ovarian cancer inhibitory sequence (OCi) that can be expressed in a eukaryotic cell, wherein said OCi encodes an inhibitory RNA for a target gene selected from FOXM1, CENPA, PLK1, CDC20, BIRC5, AURKB, CCNB1, CDKN3, BCAM-AKT2, CDKN2D-WDFY2, SLC25A6, CIP2A, CD133, ALDH1A1, CD44, SALL4, and/or PRDM16 alone or in combination, wherein said MiniVector typically lacks a bacterial origin of replication or antibiotic resistance genes, wherein said MiniVector is made by: engineering a parent plasmid DNA molecule comprising site-specific recombination sites on either side of said OCi; transforming said parent plasmid into a cell suitable for site-specific recombination to occur, under conditions such that topoisomerase IV decatenation activity is inhibited, thereby producing a plurality of catenated DNA circles, wherein at least one of the circles in each catenane is a supercoiled DNA MiniVector of typically less than about 5 kb in length; decatenating the catenated site-specific recombination products, thereby releasing the supercoiled DNA MiniVector from the catenanes; and isolating the supercoiled DNA MiniVector. |
| A method of treating ovarian cancer, comprising delivering a MiniVector or cell or composition containing same as described herein to a patient having ovarian cancer, in an amount sufficient to reduce the expression of an OCi target gene by at least 10%, at least 20% or at least 30%. |
| A treatment method as described herein, wherein said MiniVector is contained in a gel, a matrix, a solution, or a nanoparticle, and is delivered by injection or by surgery, or by other means. |
| A treatment method as described herein, wherein delivery of said MiniVector may be facilitated by electroporation, sonoporation, electrosonoporation, transfection, mechanical acceleration (gene gun, etc.), or other means, either directly into an ovarian tumor or residual tumor cells or tissue, or into cells *ex vivo* that are then returned to the patient. |
| A treatment method as described herein wherein said MiniVector is delivered to the lungs of said patient intranasally or via aerosolization; or said MiniVector is delivered intravenously, by intramuscular injection, by intraperitoneal injection; or said MiniVector is delivered topically, intravaginally, and/or rectally. |
| A treatment method as described herein wherein said MiniVector is delivered via a permanent or a temporary device, including but not limited to robot, catheter, shunt, port, arteriovenous fistula, gene gun, needle-free syringe with or without deposition technology, including but not limited to sonoporation, electroporation, electrosonoporation, etc. |
| Ovarian cancer is characterized by cancer causing FOXM1 and P53 levels and/or variants, and the method described herein can be applied to other cancers with the same characteristics. Thus, the invention includes methods of treating a cancer characterized by high FOXM1 levels, comprising delivering a MiniVector as described herein or cells or composition containing said MiniVector to a patient having a cancer characterized by high FOXM1 levels, wherein said sequence encoded by said MiniVector is a FOXM1 shRNA, wherein said FOXM1 shRNA inhibits expression of FOXM1 in said patient (or said cancer) by at least 10%, 15%, 20%, 25%, or 30%. The invention also includes methods of treating a cancer characterized by low P53 levels or P53 mutants, comprising delivering a MiniVector as described herein or cells or composition containing said MiniVector to a patient having a cancer characterized by low P53 levels or P53 mutants, wherein said sequence encoded by MiniVector is a functional P53 or a P53 stimulator, wherein said sequence reduces cancerous cell count vs. controls or increases P53 activity in said patient (or said cancer) by at least 10%, 15%, 20%, 25%, or 30%. |

### MINIVECTOR TARGETS

The p53 gene is mutated in 96% of high grade serous ovarian cancer (mucinous, endometrioid, clear cell, and undifferentiated) and negatively regulates FOXM1 (forkhead box M1), a key oncogenic transcription factor implicated in cancer-cell migration, invasion, angiogenesis, and metastasis.

The Matzuk laboratory has created the only existing mouse model (DICER-PTEN DKO) that develops high-grade serous ovarian cancer that phenocopies the spread and lethality of ovarian cancer in women (Kim 2012). In this genetically engineered mouse model, FOXM1 and its downstream targets [*i.e*., centromere protein A (Cenpa), polo-like kinase 1 (Plk1), cell division cycle 20 (Cdc20), survivin (Birc5), aurora kinase B (Aurkb), cyclin B1 (Ccnb1), and cyclin-dependent kinase inhibitor 3 (Cdkn3)] are upregulated 9.5-27.7-fold. FOXM1 is, therefore, a novel target in all p53 mutant cancers (both ovarian cancers and other cancers).

Using transcriptomic and genomic approaches, we have identified two additional novel ovarian cancer targets for novel MiniVector alone or combination therapy. These ovarian cancer-specific gene fusions are BCAM-AKT2 and CDKN2D-WDFY2.

BCAM-AKT2 is an ovarian cancer-specific fusion between BCAM (basal cell adhesion molecule), a membrane adhesion molecule, and AKT2 (v-Akt homolog 2), a key kinase in the PI3K signal pathway. BCAM-AKT2 is membrane-associated, constitutively phosphorylated, and escapes regulation from external stimuli. BCAM-AKT2 is the only fusion event proven to translate an aberrant yet functional kinase fusion and is detected in 7% of all high-grade serous ovarian cancer and it also significantly alters the PI3K/AKT pathway.

The inter-chromosomal fusion gene CDKN2D-WDFY2 occurs at a frequency of 20% among sixty high-grade serous cancer samples, but is absent in non-cancerous ovary and fallopian tube samples (Kannan 2014). The CDKN2D-WDFY2 fusion transcript was also detected in OV-90, an established high-grade serous type cell line. The genomic breakpoint was identified in intron 1 of CDKN2D and intron 2 of WDFY2 in patient tumors, providing direct evidence that this is a fusion gene. The parental gene, CDKN2D, is a cell-cycle modulator that is also involved in DNA repair, while WDFY2 is known to modulate AKT interactions with its substrates. Transfection of a cloned fusion construct led to loss of wild type CDKN2D and wild type WDFY2 protein expression, and a gain of a short WDFY2 protein isoform that is presumably under the control of the CDKN2D promoter. The expression of short WDFY2 protein in transfected cells appears to alter the PI3K/AKT pathway that is known to play a role in oncogenesis.

YM155 (a putative survivin suppressor) is an anticancer drug that is in clinical trials, but requires combination with other drugs for efficacy. The targets of YM155 were unknown. Using a proteomics screen, we identified solute carrier family 25, member 6 (SLC25A6) and cancerous inhibitor of protein phosphatase 2A, PP2A (CIP2A), as two molecular targets of YM155.

SLC25A6 is a mitochondrial membrane component of the permeability transition pore complex responsible for the release of mitochondrial products that trigger apoptosis and is a unique apoptosis target for anticancer therapy.

CIP2A, an inhibitor of tumor suppressor PP2A and a stabilizer of the MYC oncogene, is also a unique target for anticancer therapy that includes cancers that are MYC-dependent. Using shRNA delivery to OVCAR8 (human ovarian cancer cells) in culture, we have shown that knockdown of SLC25A6 and CIP2A slows ovarian cancer cell growth and triggers ovarian cancer cell death, thus validating these two targets for MiniVector gene therapy or combination therapy using MiniVectors.

FOXM1 or FORKHEAD BOX M1 was originally identified in *Drosophila,* and is a member of a family of transcription factors with a conserved 100-amino acid DNA-binding motif. FOXM1 is normally involved in cell cycle progression and is a master regulator of the DNA damage response. Although the exact mechanism remains unknown, as a proto-oncogene, FOXM1 is involved in the early stages of cancer initiation and is upregulated in many different types of cancer. These cancers include but are not limited to basal cell carcinoma, soft-tissue sarcomas, and cancers of the blood, brain, breast, central nervous system, cervix, colon, colon, rectum, kidney, liver, lung, mouth, ovary, pancreas, prostate, skin, and stomach.

A broad array of FOXM1-mediated cancers metastasizes to the lungs, including bladder, breast, colon, kidney, and prostate cancers, in addition to neuroblastoma and sarcomas. Many metastatic sarcomas are of soft-tissue origin, such as cartilage, fat, muscle, tendons, lymph vessels, blood vessels, and nerves, or from bone (Ewing sarcoma and osteosarcoma). Where metastases in the lungs occur, therapeutic nebulized MiniVectors (or intranasal application) would be employed for delivery to the lung, either alone or in combination with other therapies. MiniVector, again either alone or in combination with other therapies, may also be delivered to other locations.

### MINIVECTOR MODIFICATIONS

MiniVectors can be labeled, *e.g*., using a chemical moiety, as desired. Representative labels include fluorescent dyes, biotin, cholesterol, modified bases, and modified backbones. Representative dyes include: 6-carboxyfluorescein, 5-/6-carboxyrhodamine, 5-/6-Carboxytetramethylrhodamine, 6-Carboxy-2'-,4-,4'-,5'-,7-,7'-hexachlorofluorescein, 6-Carboxy-2'-,4-,7-,7'-tetrachlorofluorescein, 6-Carboxy-4'-,5'-dichloro-2'-,7'-dimethoxyfluorescein, 7-amino-4-methylcoumarin-3-acetic acid, Cascade Blue, Marina Blue, Pacific Blue, Cy3, Cy5, Cy3.5, Cy5.5, IRDye700, IRDye800, BODIPY dye, Texas Red, Oregon Green, Rhodamine Red, Rhodamine Green, and the full range of Alexa Fluor dyes.

Additional modifications can also include modified bases (*e.g*., 2-aminopurine, deoxyuracil, methylated bases), or modified backbones (*e.g*., phosphorothioates, where one of the non-bridging oxygens is substituted by a sulfur; methyl-phosphonate oligonucleotides).

Multiple labels, including chemical moieties and/or modified bases and/or modified backbones, can be used simultaneously, if desired. Methods of labeling nucleotides are described, for example, in Luzzietti et al. "Nicking enzyme-based internal labeling of DNA at multiple loci", in Nature Protocols (2012), vol. 7, 643-653; "Nucleic Acid Probe Technology" by Robert E. Farrell; RNA Methodologies (Third Edition), 2005, pp. 285-316; and "Enzymatic Labeling of Nucleic Acids" by Stanley Tabor and Ann Boyle, in Current Protocols in Immunology 2001 May; Chapter 10: Unit 10.10. The teachings of these references are incorporated herein by reference in their entirety.

### MINIVECTOR DELIVERY

The purified MiniVectors can be transferred into recipient cells or into a differentiated tissue by transfection using, for example, lipofection, electroporation, cationic liposomes, or any other method of transfection, or any method used to introduce DNA into cells or tissues, for instance, jet injection, sonoporation, electroporation, mechanical acceleration (gene gun, etc.), or any other method of transfer.

MiniVector may be delivered in a gel, a matrix, a solution, a nanoparticle, a cell, or other means directly into an ovarian tumor or residual tumor cells or tissue, or into cells *ex vivo* that are then returned to a patient. Typically, *in vivo* studies use injection or surgical introduction, but any method can be used *ex vivo.* The term "cell" includes Car T cells or any cell therapy.

Delivery solutions can be aqueous solutions, non-aqueous solutions, or suspensions. Emulsions are also possible. Delivery solutions can be magnetic, paramagnetic, magnetically resistant, or non-magnetic. Saline is a preferred delivery solution. The MiniVector therapy could optionally be lyophilized.

The preferred carrier medium for the MiniVector can vary depending on whether it is delivered systemically or locally. The complexity of the carrier could vary as a result. Systemic carriers can be more complex given the need for enlarged circulation times and the need to resist a variety of *in vivo* processes which might prematurely degrade the carrier and the MiniVector (e.g. opsonization).

Systemic carrier mediums can have varied in size vs. locally-delivered carriers. Further, the size of the carrier medium can change due to an array of stimuli (e.g. charge, enzyme availability, magnetic field, etc.). Some research suggests that the optimal particle size during the "systemic" phase is approximately 100 nm and during the "local" accumulation phase is approximately 40 nm.

Components that might be included in a carrier medium include but are not limited to: exosomes, biomimetic proteolipid vesicles (e.g., "Leukosomes"), Lipidoids, porous silicon micro and/or nanoparticles, dendrimers, spermine, spermidine, polyethylenimine (PEI), saline, liposomes, phospholipids, lipids - cationic or otherwise, lipoplexes, nanoemulsions - cationic or otherwise, nano or micro-porous silicate nanoparticles, nano or micro-porous silicate microparticles, nano or micro-porous gold nanoparticles, nano or micro-porous gold microparticles, chitosan, cholestoral, hydrazone activated polymers, zwitterionic polymers or co-polymers, amphiphilic polymers or co-polymers, polyplexes modified with PEG or HPMA, polymethacrylate, biodegradable polyesters (e.g. poly-(DL-Lactide) aka "PLA" or poly-(DL-Lactide-coglycoside) aka "PLGA"), amino acid-derived polymers, biodegradable amino acid polymers, biodegradable amino acid polymers configured in a star or dendritic fashion, and microbubbles. Hybrids or combinations of the listed materials and others are possible.

Optionally the carrier medium can employ a core-shell morphology where the core and the shell optionally have multiple layers and said layers have different charge and contact angle (i.e. hydrophobic, hydrophilic, amphiphilic) and said materials optionally degrade or change due to variations in pH, temperature, shear energy, light, time, the presence of enzymes and the like. Said particles can be conjugated with a broad array of agents which affect shape, size (e.g. shrinking or swelling), immune system activation (e.g. adjuvant), endocytosis, nuclearization and the like. Particles can be nanosized or micro-sized. Such structures can optionally be lamellar. Their interfaces can be graded.

The carrier medium or its components can be self-assembled.

Carrier mediums can "open" to facilitate delivery of the MiniVector when subjected to ultrasound, magnetic field, redox, light, enzymes and the like.

A carrier medium can be a multi-layer polymer encapsulated particles where the layers degrade over time.

To reduce the propensity of the body to degrade the employed carrier medium (e.g. via opsonization and phagocytic uptake), it can be optionally coated or treated with a "stealth" material. Stealth materials are frequently electrostatically neutral and/or hydrophilic. Examples of passive "stealth" materials include but are not limited to polyethylene glycol ("PEG"), polyvinyl alcohol ("PVA"), polyglycerol, poly-N-vinylpryrrolidone, polyozaline, and poly[N-(2 hydroxypropyl)methacrylamide].

Appreciating that stealth materials can reduce the carrier medium's interaction with cell surfaces and thus cellular uptake, they can be engineered to separate ("cleave") from the carrier medium when subjected to stimuli. Examples of such stimuli include but are not limited to light, ultrasound, magnetic field, pH, redox, or enzymes.

The carrier medium can employ targeting agents (frequently attached as ligands) which enhance accumulation at target sites. Targeting agents include but are not limited to antibodies, aptamers, peptides, and small molecules that bind to receptors on the cell.

Peptide-types include but are not limited to those which exhibit high affinity for a targeted cell surface receptor (e.g. cell targeted peptides or "CTPs" such as arginine-glycine-aspartic acid aka "RGD" tripeptide). Other peptides can exhibit properties which afford the ability to non-specifically interact with cell surfaces and enhance cell entry (e.g. protein transduction domains or "PTDs" and cell penetrating peptides or CPPs).

Example non-peptide targeting agents include cholera toxin B, folic acid, low-density lipoprotein, nicotinic acid, riboflavin, and transferrin (e.g. Arrowhead Pharmaceutical's CALAA-01).

Solutions of all types may be combined with other phases such as gasses for purposes of delivery. A typical example would be for the purpose of atomization and more specifically control of droplet size and droplet size distribution.

MiniVector delivery can be facilitated by an *ex vivo* or *in vivo* device that meters out delivery quantities locally or systemically, but preferably locally. Said devices can control or influence other desired properties such as temperature, pH, shear, and dispersion uniformity. Said devices will likely comprise microelectromechanical (MEM) or nanoelectromechanical (NEM) components. Said devices could afford multiple purposes ("Combination Devices") *ex vivo* or *in vivo.* Functions afforded by a Combination Device could include therapeutic dispensing and optionally therapeutic atomizing, pH control, heating, cooling, magnetic potential control, sensing of these and other activities, and wireless communication amongst others.

Solutions of MiniVectors can be delivered locally into the peritoneal cavity via a needle, a minimally invasive surgical device, or a surgical device during surgery more generally. Said surgical devices could comprise an atomizer and the solution of MiniVectors could optionally be atomized. Atomization could be achieved via control of nozzle aperture, pressure, or the introduction of a second phase *(e.g.,* a gas). Droplet size and size distribution could be controlled in similar fashion.

MiniVector therapies could be stored in powder form, gel form, as an emulsion, or as a solution, or as a precipitate under alcohol. To maximize the shelf-life of any MiniVector therapy a variety of preservatives can be employed. Example preservatives include but are not limited to ethyleneglycol-bis(2-aminoethylether)-N,N,N',N'-tetraacetic acid ("EGTA"), ethylenediaminetetraacetic acid ("EDTA"), a nuclease inhibitor, a protease inhibitor, or any other chelating agent.

### COMBINATION THERAPIES

To improve the efficacy of MiniVector-based therapies, they may be administered in combination with other MiniVectors or with other FDA approved therapies. Thus, MiniVectors can be administered before, concurrently with, and/or following treatment with other MiniVectors, small molecule drugs, peptides, antibodies, siRNA, minicircles, ministrings, plasmids, viruses, surgery, or radiation, or any combination and/or timing of administration of two, three or more of these individual approaches.

Combination therapy is regularly used in cancer treatment because of the heterogeneity and complexity of the disease, and the tendency of cancer cells to become resistant to certain drugs. There are many potential benefits to using such an approach with MiniVectors. Primarily, although resistance to current frontline small molecules and radiation occurs, the genetic basis of cancer *(e.g.,* p53 downregulation) remains. Therefore, although it remains a theoretical possibility, there is no reason to believe that resistance to gene therapy would emerge-indeed, to date this has never happened. Therefore, a MiniVector therapy should be able to block growth of any chemoresistant or radiation-resistant cancer and the genetic basis for the resistant tumors are easily checked by sequencing prior to MiniVector treatment. To the extent that the adjunct therapy and the MiniVector target different pathways, this should increase the success of cancer eradication by targeting two different pathways. Furthermore, because of the increased efficacy, combination therapy may allow lower doses of each therapy to be used, and consequently lower toxicity and reduced side effects.

PARP inhibitors have shown great promise for ovarian cancer treatment and a number of drugs that target this protein have recently been approved. PARP is involved in the repair of single-strand breaks in DNA. When PARP is inhibited these single-strand breaks become double-strand breaks and must be repaired by homologous recombination. A significant proportion of ovarian cancers have impaired homologous recombination. The inability of the cells to repair the DNA breaks ultimately leads to death of the cancer cells. PARP inhibitors have been assayed in combination with chemotherapy or radiation because the inhibition of DNA repair by PARP inhibitors should enhance the efficacy of these treatments.

One potential combinatorial therapy will be the concurrent administration of a MiniVector encoding an shRNA to FoxM1 and a Poly ADP-ribose polymerase (PARP) inhibitor, for example, Rucaparib, Niraparib, Lynparza, Olaparib, and/or Talazoparib. PARP inhibitors we can thus target two distinct characteristics of the cancer cells. This combinatory approach may lack the synergistic power of PARP inhibitors combined with chemotherapy or radiation, but should have lower toxicity and also a reduced incidence of resistance. The most likely application for such an approach is to target any remaining cancer cells following surgery. The lower toxicity and reduced incidence of resistance should be useful for the prolonged treatment necessary to eliminate the cancer cells and prevent relapse.

In an alternative approach, MiniVectors and PARP inhibitors could be combined with chemotherapy and/or radiation for the purpose of maximizing patient longevity, and minimizing treatment-induced toxicity and drug resistance. It is understood that quantities of each therapy type would be optimized combinatorially in animals and humans for the purpose of maximizing net benefit.

Chemotherapy agents available for combination therapy with MiniVectors include but are not limited to Paclitaxel, Capecitabine, Cyclophosphamide, Etoposide, Gemcitabine, Topotecan, Doxorubicin, Cisplatin, Carboplatin, Vinorelbine, Ifosfamide, Etoposide, Fluorouracil, Docetaxel, or combinations thereof. Tyrosine kinase inhibitors, such as Pazoparib, can also be combined with MiniVectors.

Radiation therapies available for combination with MiniVectors include but are not limited to proton therapy, two-dimensional proton therapy, three dimensional conform radiation therapy, brachytherapy, intensity modulated radiation therapy, image guided radiotherapy, stereotactic radiation, radio surgery, orthovoltage radiation, electron radiation, or combinations thereof.

Biological approaches, including hormone therapies, available for combination with MiniVectors include but are not limited to Aromassin, Femara, Arimidex, Megace, Farletuzumab, Tamoxifen, Rucaparib, Niraparib, Olaparib, and/or Talozaparib.

Surgical procedures that can be combined with MiniVectors include but are not limited to tumor debulking, oophorectomy, salpingectomy, hysterectomy, omentectomy, and "second look" surgery where the efficacy of assorted treatments is interrogated *in vivo.*

Another ovarian cancer therapy approach available to be combined with MiniVectors is that of tumor ablation. During tumor ablation micro- or nano-sized particles of varied composition are delivered adjacent to cancers. The particles typically preferentially ablate tumors when subjected to thermal energy, radiofrequency, ultrasound, microwave and/or a cryoablation source, amongst others. The acting particles often concurrently protect or minimize damage to healthy tissues adjacent to the tumor. Examples of candidate particles suitable for ablation include but are not limited to gold (as undergoing commercialization by NanoSpectra), platinated nanoparticles, copper sulfide, and polydopamine. MiniVectors could be delivered by the methods described herein before, during, or after ablation therapies.

Stem cell-derived therapies afford great promise in a variety of medical domains including ovarian cancer. Stem cell-derived cell therapy approaches frequently augment patients' immune systems by awakening the body to the presence of assorted cancers or cancer-driving mutations. In one embodiment, adult stem cells are repurposed to express a T-cell receptor known to NY-ESO 1. When the repurposed stem cells are re-injected into the patient a portion of said cells expand and self-renew. These surviving repurposed T-cells alert the body to the presence of certain ovarian cancer types and thereby initiating a series of processes that result in the destruction of the tumor cell. Stem cell-derived immunotherapy approaches and others could be combined with MiniVector-derived therapies.

Another combination therapy approach would be to deliver multiple MiniVectors, each encoding a different sequence. Delivering MiniVectors compositions that inhibit more than one target may produce a synergistic effect and be more effective than a single-target MiniVector. In addition, delivering multiple MiniVectors against targets on different pathways may reduce toxicity and likelihood of resistance. Both approaches will be tested for feasibility.

A key context in which MiniVectors could be used following other therapies is ovarian cancer tumor recurrence. More than 50% of ovarian cancer patients that undergo surgical tumor resection experience recurrence and most of these recurring cancers are resistant to the previous chemotherapeutic or radiation treatment(s). There are only limited drugs or treatments available such that there remains no choice left but to stop treatment. In addition, the human body has limited capacity to undergo repeated surgical, chemical, and radiation treatments; however, ovarian cancer recurrence is frequently fatal, particularly for high grade serous ovarian cancer. Although recurring ovarian cancer is typically resistant to the previous treatments, the genetics behind the cancer phenotype *(e.g.,* p53 downregulation) remains. Therefore, MiniVectors can still be used in these cases. Furthermore, the cassette encoded by MiniVectors (see **FIG. 2**) can be changed repeatedly and treatments continue.

MiniVectors afford the ability to treat ovarian cancer patients repeatedly and perhaps on a long-term basis, even when the patient has undergone other treatments (that may or may not have been combined with MiniVector therapies). Reasons for the possibility of repeated MiniVector treatment include the fact that they can be delivered locally vs. systemically, their lack of toxicity as a consequence of their small size and their "naked" DNA composition (limited cytotoxicity and immune response), the lack of unwanted delivery or integration (high shear strength of the closed circular particles), and the facile switching of MiniVector cassettes.

These attributes in combination afford the ability to modify ("tune") MiniVector composition and its cassette to address both spatial and temporal tumor heterogeneity and to mitigate or obviate tumor recurrence. It is specifically possible to employ said tuned MiniVectors either alone, combinations of MiniVectors, or in combination with other therapies *(e.g.,* small molecule, other gene therapy approaches, cell therapy agents, other MiniVectors, and biologics).

### PERSONALIZED MINIVECTOR THERAPIES

Although our proof-of-concept work will continue to proceed with commercial sequences that target common mutations, we contemplate developing personalized target sequences for each patient. Cancer is highly heterogenous in nature. No two cancers are identical and cancer cells may even vary within the same patient. The DNA sequence and gene expression profiles of an individual patient's cancer can be readily determined through high-throughput DNA sequencing, microarrays, qPCR, RNA-Seq, and other methods on patient tissue samples. These tests reveal which sequences and/or gene product(s) are present or absent, and which genes are abnormally expressed in the cancer cells, so that a custom MiniVector can be developed encoding targets specifically tailored to a particular patient. This type of approach can be readily modified as needed and according to treatment outcomes by altering one or more of the sequences encoded on the personalized MiniVector or MiniVectors.

In ovarian cancer, 7% of patients with high-grade serous ovarian cancer have the gene fusion BCAM-AKT2 and 20% have the gene fusion CDKN2D-WDFY2. These gene fusions are absent from normal cells and therefore these targets are unique to this subset of ovarian cancers. For a personalized therapy approach, individuals presenting these fusions would be treated with sequences that target these proteins specifically.

An additional example of personalization of MiniVector/MiniVectors treatment is to target specific isoforms of a protein that are present in certain cancer patients. The rationale for which isoform is targeted will rely on those determined from ovarian cancer tissue samples. Once the isoform(s) is determined, shRNA(s) can be designed to specifically knockdown that isoform(s), based on identified unique sequences of exons included in the isoform. FoxM1b and FoxM1c, for example, are two isoforms of FoxM1 that are upregulated and transcriptionally active in many cancers, including ovarian cancer. FoxM1b has a greater transforming potential than FoxM1c. FoxMla is not transcriptionally active in cancer cells. As part of alternative splicing, FoxMlb lacks exon Va, a stretch of 15 amino acids, whereas FoxM1a and FoxM1c contain this exon. An shRNA construct will be designed to specifically target the FoxM1b isoform using the unique sequence that connects exons IVa and VIa. This personalization will allow specific knockdown of FoxM1b for treatment and will not target the FoxM1a or FoxM1c isoforms.

### MODEL OVARIAN CANCER STEM CELL LINE

Although women with ovarian cancer initially respond well to surgical debulking and chemotherapy, there is a high cancer recurrence rate that is hypothesized to arise secondary to the chemoresistant population of cancer stem cells (CSC). We have identified, for the first time, an ovarian CSC-like cell line (called OV90) that tends to form spheroids (spherical 3 dimensional cultures) under standard culture conditions, greatly expressing stem-like markers Prominin 1 (CD133), Aldehyde Dehydrogenase 1 Family Member A1 (ALDH1A1), CD44 Molecule (CD44), Spalt Like Transcription Factor 4 (SALL4), and PR/SET Domain 16 (PRDM16), as well as FOXM1. OV90 cells are resistant to many National Cancer Institute oncology library compounds.

The OV90 line will allow us to test the effects of novel treatments *in vitro* and *in vivo* (in cell line xenograft models) and make advances toward treatment of cancer patients. Because of the role of FOXM1 in sternness, the OV90 cells will be particularly useful for testing MiniVectors or combination therapies involving MiniVectors that target FOXM1 for their ability to kill these ovarian cancer stem cells. Ovarian cancer stem cell markers (e.g., CD133, ALDH1A1, CD44, SALL4, and PRDM16) are also potential targets for MiniVector therapy.

### PROOF OF CONCEPT FOXM1 EXPERIMENTS

For our initial work, we tested FOXM1 shRNA (targeting 5'-ATAATTAGAGGATAATTTG-3') in plasmid vectors against the ovarian cancer cell line OVCAR8. In these initial experiments, we were able to show ∼25% knockdown after 72 hours post-transfection and significant cancer cell inhibition **(****FIG. 5A****).** In a control non-cancerous cell culture line, 293T cells, we achieved 60% knockdown of FOXM1 (the gene is not overexpressed in non-cancer cell lines, so there was a lower amount to start with) but there was no inhibition of growth **(****FIG. 5B****).**

Since that early experiment, we have also confirmed proof of concept using MiniVectors. FOXM1 shRNA (targeting 5'- ATAATTAGAGGATAATTTG-3') in MiniVector was tested against the ovarian cancer cell line OVCAR8 **(****FIG. 6A****).** In these initial experiments, we were able to show between ∼16 to 48% knockdown of FoxM1 when MiniVectors were delivered in a dose response manner, 72 hours post-transfection. In a control non-cancerous cell culture line, 293T cells, we achieved between 25 to 50% knockdown of FOXM1 with no inhibition of cell growth **(****FIG. 6B****).** Therefore, we have a good indication that our approach using MiniVector therapy will be far safer for use in humans.

An 11.8 kb plasmid (obtained from Dharmacon, Inc.) or 0.4 kb MiniVector encoding shRNA against FOXM1 were delivered via Lipofectamine 3000 to human high grade serous carcinoma (HGSC) OVCAR8 cells. As controls, an 11.8 kb plasmid encoding a "non-targeting control" (pCMV-NTCshRNA) (also from Dharmacon, Inc.) or a 0.4 kb MiniVector encoding shRNA against Green Fluorescent Protein (GFP) were used for off-target effects.

Knockdown of FOXM1 was assessed three days post-transfection using quantitative real time polymerase chain reaction (qRT-PCR), in which the plasmid and MiniVector encoding the FOXM1 shRNA showed reduced expression of FOXM1. At the same mass equivalency of plasmid and MiniVector (*i.e*., 0.35 µg), FOXM1 expression was reduced by 17% and 49%, respectively, which is an 85-fold improved knockdown efficiency for the MiniVector given the size difference of these two vectors (**FIG. 7A**).

At a 3-fold higher dosage, MiniVector achieved a 60% reduction in FOXM1 expression compared to the control experiments (**FIG. 7A**). In addition, cell growth was monitored at 3, 7, and 12 days post-transfection with the same plasmids and MiniVectors above and assessed with flow cytometry. Cell death for the OVCAR8 cells treated with FOXM1 shRNA vectors reached ∼38% and ∼76% for plasmid and MiniVector, respectively, compared to untransfected cells when averaging the data from all three days (**FIG. 7B**). This is a 60-fold improved cell killing for MiniVector given the size difference with the plasmid. Images from day 11 post-transfection show hardly any cells in the MiniVector-treated sample versus the control (**FIG. 8**)

In another experiment, the same doses of plasmid and MiniVector encoding shRNA against FOXM1 as described in the experiments for **FIG. 7** above were transfected into human embryonic kidney 293T cells, a non-cancer cell line. As shown in **FIG. 9A**, the same mass equivalency of plasmid and MiniVector (*i.e*., 0.35 µg) showed a 54% and 87% reduction, respectively, of FOXM1 expression by qRT-PCR compared to the control experiments; this is a 48-fold improved efficiency for the MiniVector given the size difference of the two vectors. While there was a dramatic knockdown of FOXM1, the 293T cells remained viable and cell growth numbers were not reduced the same as OVCAR8 cells (<5%) as monitored by flow cytometry for both plasmid and MiniVector transfections (**FIG. 9B**).

Given the successful knockdown of FOXM1 in cell culture, and lack of toxicity evidenced in non-cancerous cells, the plasmid and MiniVector encoding the shRNA against FOXM1 were tested for efficacy in a high grade serous carcinoma (HGSC) xenograft mouse model. For this model, OVCAR8 cells were stably transfected with the plasmid pGL4.EF1a-*luc*2/Hygro.gcs, which encodes firefly luciferase (*luc2*) and is maintained using G418 selection. The FOXM1 shRNA plasmid and MiniVector, as well as negative control MiniVectors (empty and shRNA against GFP), were delivered via electroporation (using the Neon Electroporation System) into bioluminescent OVCAR8 cells.

Transfected OVCAR8 cells were allowed to recover for 24 hours in culture and then were injected intraperitoneally (IP) into female non-obese diabetic severe combined immuno-deficient (NOD-SCID) mice. Two weeks later, mice were injected IP with the luciferase substrate D-luciferin at 10 mg/ml, anesthetized, and imaged ∼5 minutes following injection at the luciferin peak fluorescence using an IVIS Spectrum *In Vivo* Imaging System. Images in **FIG. 10** show that the FOXM1 shRNA MiniVector prevented HGSC xenograft growth *in vivo.* While the mouse treated with plasmid died at day 23, the FOXM1 shRNA MiniVector-injected mouse lived 61% longer; however, while this mouse was not demonstrating any obvious distress, it was euthanized on day 37.

### PROPHETIC FOXM1 EXPERIMENTS

The following prophetic examples can apply equally to all cancers described herein. For brevity however, they are written relative to ovarian cancer. Other described cancers could be substituted provided the appropriate cancer specific cell line (see Table 6) and animal model (*i.e*., xenografted or patient-derived xenografted mice) are employed. Also, while FOXM1 is relevant to all p53 related cancers described herein, other named targets specific to a relevant cancer (*e.g*., ovarian cancer) can be substituted or added to the FOXM1 example in the prophetic experiments without limitation. In addition, although *e.g*., FOXM1 may be the primary target, similar effects may be obtained by targeting up- or downstream genes in the same pathway.

Knockdown efficiency of the *de novo* shRNAs will be validated using synthetic small interfering RNAs (with the same RNA sequence as the shRNA transcripts) that will be transfected into different cell lines *(e.g.,* OV90 or OVCAR8 cell lines for ovarian cancer; see below) using lipofection, electroporation, sonoporation, or any other method of nucleic acid delivery for cell culture. Knockdown will be assayed using SYBRTM Green PCR Master mix to measure levels of the target mRNA in cell lysates. Knockdown efficiencies of the siRNAs will be compared to validated shRNA sequences (encoded on pGIPZ plasmid vectors) obtained from Dharmacon (see Table 1).

shRNA sequences that demonstrate effective levels of knockdown efficiency (>10% reduction in mRNA levels as determined by quantitative RT-PCR (Q-RT-PCR) analysis and/or >10% reduction in protein levels as determined by western blot analysis) will be cloned between the *att*B and *att*P recombination sites on the MiniVector, generating parent plasmid using standard, well-established molecular cloning techniques. MiniVectors are the products from an intramolecular recombination reaction as described above and below. In the most basic embodiment, the resulting MiniVector will comprise elements include a promoter, the therapeutic sequence, a terminator, and the hybrid site from recombination (*att*L or *att*R). Accessory sequences can be added to improve efficiencies, such as enhancers, DNA targeting sequences (DTS's), etc. (see Tables 2-5).

### MINIVECTOR SYNTHESIS

MiniVectors are generated using engineered *Escherichia coli* strains (examples include but are not limited to LZ31 and LZ54), in which a small aliquot of this strain is transformed with the relevant parent plasmid, growth, and then is used to inoculate a fermenter containing modified terrific broth medium. Cells are grown at 30°C with maintaining the pH at 7 and the dissolved oxygen concentration above 60%. Once cells have reached mid-exponential phase, λ-Int expression is induced by shifting the culture to 43°C. Norfloxacin is added to prevent decatenation by topoisomerase IV, and the culture is shifted down to 28°C to allow recombination to proceed for about an hour (1-4 hrs). Cells are harvested by centrifugation.

MiniVectors are purified by first resuspending the cells in buffer and lysozyme, and further lysed using alkaline lysis. Nucleic acids are precipitated by isopropanol and treated with RNase A and Proteinase K. Parent plasmid is removed through precipitation with polyethyleneglycol. MiniVectors are further purified from their parent plasmid using anion exchange purification kits and gel filtration. Endotoxin is removed using commercially available purification kits.

### FOXM1 SHRNA EVALUATION

For ovarian cancer, an ovarian cancer stem-cell like cell line (OV90) or another ovarian cancer cell line *(e.g.,* OVCAR8) will be transfected with MiniVectors encoding a single or a combination of multiple shRNAs for the same target or for different targets with the purpose of assessing the effect of single or combination shRNA for blocking cancer growth. As described above, MiniVectors will encode one or multiple shRNA sequences that have been screened to avoid off-target effects based upon sequence homology. When using more than one shRNA against the same or multiple different target genes, combinatorial knockdown of the target gene or genes may increase either the overall knockdown efficiency or the inhibition of cancer cell growth. Consequently, lower doses of each shRNA-encoding MiniVector may be required with the added benefit of minimizing potential off-target effects of each specific shRNA sequence.

To test knockdown of the MiniVector-encoded shRNA gene targets *in vitro,* cells will be plated and transfected with LipofectamineTM3000 transfection reagent once they reach 80% confluency. We may also use other methods of DNA delivery in culture (electroporation, sonoporation, *etc.).* 24 and 72 hours post-transfection, cells will be trypsinized and harvested for RNA extraction and cDNA synthesis. Q-RT-PCR will be conducted to quantify knockdown of FOXM1 or other target genes using SYBRTM Green PCR Master mix. We anticipate knockdown efficiencies to show a >10% reduction in mRNA levels as determined by Q-RT-PCR analysis and/or >10% reduction in protein levels as determined by western blot analysis. The effects of these knockdown levels will be further measured by assessing the phenotype of the cultured cancer cells.

The phenotype from the knockdown of FOXM1 will be assessed in culture by measuring cell apoptosis, cell cycle arrest, and cell proliferation. We will use flow cytometry and commercially available kits to measure these variables. We predict that the therapy or combination therapies that result in sustained knockdown of the targets will have the best ability to block cancer growth.

Off-target effects and cytotoxicity resulting from the knockdown of FOXM1 will be measured concurrently with the experiments outlined above by transfecting a non-cancer cell line (*i.e*., 293T cells). mRNA from cell lysates of cancer cells, and/or 293T cells will be used to do microarray analysis or RNAseq to further confirm the lack of off-target effects of the therapies. Cytotoxicity will be measured using cell viability and apoptosis assays. Any potential shRNA candidates that display any deleterious level of off-target effects or cytotoxicity will not be pursued *in vivo.*

An alternative therapy to block growth of cancer cells will be transfection with MiniVectors encoding genes that promote apoptosis *(e.g.,* p53, p16, p21, p27, E2F genes, FHIT, PTEN, or CASPASE). In the most basic embodiment (see **FIG. 2**), the resulting MiniVector encoding such genes will have a promoter, the apoptosis-promoting sequence, a terminator, and the hybrid recombination site (*att*L or *att*R). The benefit of this approach will be assessed by measuring cell apoptosis, cell cycle arrest, and cell proliferation in the target cancer cells.

MiniVectors encoding the best shRNA candidates for single and combinatorial therapies against FOXM1, and with demonstrated efficient knockdown and corresponding phenotype in cell culture, will be pre-clinically screened *in vivo* first in cell line xenograft and then patient derived xenograft mouse models to assess cancer cell-death (via apoptosis or another mechanism) or slowdown of cancer cell growth, and also to further optimize and formulate treatment therapies against different cancers, some of which are based on metastatic models.

Bioluminescent cell lines for cell line xenografting will be generated by stably transfecting various cancer cell lines with a vector encoding a Luciferase reporter (pGL4.51) using lipid transfection or any other method of nucleic acid delivery. Selection of cells stably transfected with pGL4.51 will be achieved with the antibiotic G418. Mice will be injected with D-luciferin (the luciferase substrate) by intraperitoneal (IP) injection. Bioluminescence from the luciferase enzyme will allow *in vivo* imaging to be used to detect and quantify any changes in the size of the tumors. Alternative imaging methods include making a stable cell line with a different fluorescence-encoding reporter plasmid *(e.g.,* GFP, RFP, *etc.).*

Cells stably expressing the luciferase or fluorescence reporter will be transfected *in vitro* with MiniVectors encoding the shRNA candidates (using transfection or any other method of nucleic acid delivery), followed by IP injection of the transfected cells to female immunodeficient eight-week athymic nude (Foxnlnu) mice (or other appropriate mouse models) to generate the cell line xenografts.

Growth or proliferation of these cancer cells as well as the size of the tumors in mice will be tracked in real-time by *in vivo* bioluminescence (after IP injection of the D-luciferin substrate) or fluorescence imaging. Negative controls in these experiments will be vehicle only (no MiniVector) or MiniVectors encoding control (for example, scrambled validated control sequences) shRNA sequences. We predict that shRNAs shown to result in efficient knockdown of the target genes will successfully attenuate growth of the tumors relative to mice xenografted with vehicle only or with MiniVectors encoding control shRNA sequences. At the completion of the bioluminescence or fluorescence studies, mice will be sacrificed and dissected, and tumors evaluated.

Tumors as well as other organs will be harvested for gene expression and histological analysis. Q-RT-PCR and western blot will be done in tumor homogenates to quantify knockdown of cancer targets. Histology of the organs will be assessed, and any cytotoxicity or off-target effects of the therapy will be noted. We will re-formulate the therapy if needed.

Bioluminescent cancer cells will be injected into mice to generate a xenograft mouse model. MiniVectors will be delivered subsequently either immediately or by varying the time following tumor engraftment by IP injection and any changes in tumor size will be quantified using bioluminescent imaging compared to control xenograft mice treated with control MiniVectors or untreated. Other possible routes of *in vivo* MiniVector delivery in mice include intranasal, intravenous (tail vein, face vein, or other), intramuscular, topical applications or other methods to reach tumors. Either naked DNA MiniVectors or the use of delivery vehicles (lipofectamine, lipid polymers, etc.) will be tested, and optimized if needed, by measuring tumor reduction and knockdown of the target. In the case where metastases to the lungs are being interrogated, nebulized (or intranasally instilled) MiniVectors would be employed for delivery to the lung alone or in combination with other delivery mechanisms and locations.

Dosage, treatment frequency, as well as duration of the therapy will be assessed by measuring and monitoring tumor size, measuring knockdown of the target mRNAs, and assessing toxicity.

### MINIVECTORS WITH SPECIFIC SHAPES

MiniVector DNA backbone sequence can be modified to engineer DNA sequence and supercoiling-dependent bends to affect DNA 2-dimensional (if planar) or 3-dimensional shape. Geometries such as, but not limited to, rod-shaped, two, three, four, and five or more-leafed clover-shaped, triangle-shaped, square-shaped, rectangle-shaped, trapezoid-shaped, kite-shaped, both regular and irregular pentagon-shaped, hexagon-shaped, other polygon-shaped, star-shaped, disc-shaped, sphere-shaped, ellipse-shaped, cylinder-shaped, cone-shaped, crescent-shaped, obelisk-shaped, tetrahedron-shaped, hexahedron, octahedron-shaped, dodecahedron-shaped, icosahedron-shaped, pointed shapes, shapes that mimic viral capsids, hybrids of these shapes, convex and concave versions as well of each of these geometries, and the like can be engineered to improve transfection or preferentially target one cell type over another. MiniVector shapes may change or be induced over time or with specific condition (encounter with proteins, salts, cell compartment-specific environment, temperature, pH, etc.) from one to another shape.

### ADDITIONAL EXPERIMENTS

Novel therapeutic shRNA sequences (at least 5) against each of the primary targets, FOXM1, BCAM-AKT2, and CDKN2D-WDFY2, will be designed using freely available, open access, algorithms (*e*.*g*., siRNA Wizard™ Software, siDESIGN Center, etc.) and then screened for off targets effects using NCBI-BLAST. Alternatively, commercially available sequences can be used for initial proof of concept work.

Knockdown efficiency of the *de novo* shRNAs will be validated as described above, and shRNA sequences that demonstrate effective levels of knockdown efficiency will be cloned between the attB and attP recombination sites on the MiniVector, as above. The MiniVectors will then be tested in OV90 or OCVAR8.

Knockdown (or increases) in targets can be measured by measuring mRNA levels or protein activity in e.g., biopsy or patient fluids or in cell culture or in animal models. The phenotype from the knockdown of targets, such as FOXM1 or BCAM-AKT2 or CDKN2D-WDFY2, may also be assessed in cell culture by measuring cell apoptosis, cell cycle arrest, and cell proliferation. We will use flow cytometry and commercially available kits to measure these variables. We predict that the therapy or combination therapies that result in sustained knockdown of the targets will have the best ability to block cancer growth.

Off-target effects and cytotoxicity resulting from the knockdown of FOXM1 or BCAM-AKT2 or CDKN2D-WDFY2 will be measured concurrently with the experiments outlined above by transfecting a non-ovarian cancer cell line (*i.e*., 293T cells). mRNA from cell lysates of OV90, OVCAR8, and/or 293T cells will be used to do microarray analysis or RNAseq to further confirm the lack of off-target effects of the therapies. Cytotoxicity will be measured using cell viability and apoptosis assays. Any potential shRNA candidates that display any deleterious level of off-target effects or extreme cytotoxicity will no longer be pursued *in vivo.*

A therapy to further sensitize cancer cells for shRNA treatment will include the co-transfection with MiniVectors-encoding genes that promote apoptosis (*e.g*., p53, pl6, p21, p27, E2F genes, FHIT, PTEN, or CASPASE). In the most basic embodiment, the resulting MiniVector-encoding such genes will have a promoter, the apoptosis-promoting sequence, a terminator, and the hybrid recombination site (*att*L or *att*R). The benefit of this approach will be assessed by measuring cell apoptosis, cell cycle arrest, and cell proliferation in OV90 and/or OVCAR8 cells.

MiniVectors encoding the best shRNA candidates for single and combinatorial therapies against FOXM1 or BCAM-AKT2 or CDKN2D-WDFY2, and with demonstrated efficient knockdown and corresponding phenotype in cell culture, will be pre-clinically screened *in vivo* in a bioluminescent or fluorescent cell-line xenograft mouse model (mice injected with bioluminescent or fluorescent human high-grade serous ovarian cancer cell lines) to assess cell-death (via apoptosis or another mechanism) or slowdown of cancer cell growth, and also to further optimize and formulate treatment therapies against ovarian cancer.

Bioluminescent cell lines for xenografting with be generated by stably transfecting ovarian cancer cells (OVCAR8 or OV90) and non-ovarian cancer cells (U2OS) with a vector encoding a Luciferase reporter (pGL4.51) using lipid transfection or any other method of nucleic acid delivery. Selection of cells stably transfected with pGL4.51 will be achieved with the antibiotic G418. Mice will be injected with D-luciferin (the luciferase substrate) by IP injection. Bioluminescence from the luciferase enzyme will allow *in vivo* imaging to be used to detect and quantify any changes in the size of the tumors.

Cells stably expressing the Luciferase reporter will be first transfected *in vitro,* with MiniVectors-encoding the shRNA candidates (using lipid transfection or any other method of nucleic acid delivery), followed by IP injection of the transfected cells to female immunodeficient eight-week athymic nude (Foxnlnu) mice to generate the xenografts. Growth or proliferation of these cancer cells as well as the size of the xenograft in mice will be tracked in "real-time" by *in vivo* bioluminescence imaging after IP injection of the D-luciferin substrate. We predict that shRNAs shown to result in efficient knockdown of the target genes will successfully attenuate growth of the tumors, relative to mice xenografted with MiniVectors encoding control shRNA sequences. At the completion of the bioluminescence studies, mice will be sacrificed and dissected to ensure that tumor size corresponds to bioluminescence data.

Tumors as well as other organs will be harvested for gene expression and histological analysis. Quantitative real-time PCR and western blot will be done in tumor homogenates for validation of the cancer targets *in vivo.* Histology of the organs will be done to assess cytotoxicity or any off-target effects of the therapy. This will allow us to re-formulate the therapy if needed.

Bioluminescent ovarian cancer cells will be injected into mice to generate a xenograft mouse model. MiniVectors-will be delivered separately by IP injection and any changes in tumor size will be detected and quantified using bioluminescent imaging and compared to control xenograft mice treated with control MiniVectors. Other routes of *in vivo* delivery in mice (intranasal, tail vein injection, intramuscular, topical applications to tumors) as well as delivery vehicles (naked DNA, lipofectamine, lipid polymers, *etc.)* will be tested, and optimized if needed, by measuring tumor reduction and knockdown of the target.

Dosage, treatment frequency, as well as duration of the therapy will be assessed by measuring and monitoring tumor size and also by measuring knockdown of the target mRNAs, and toxicity.

Note that if the therapeutic sequence is shRNA, the promoter will likely be U6 or H1 or another promoter recognized by mammalian RNA polymerase III. If said therapeutic sequence is a gene (p53, p16, p21, p27, E2F genes, PTEN, caspase, or another apoptosis inducing gene), the promoter will be CMV, EF1α, or another promoter for mammalian RNA polymerase II.

| **Table 1.** Therapeutic sequences to be encoded on MiniVector | | | | |
|---|---|---|---|---|
| SEQ ID NO | Gene | Description | Dharmacon Cat. No. | Mature Antisense |
| 1. | AKT2 | RAC-beta serine/threonine-protein kinase (gene AKT2) P31751 | V2LHS_237948 | AAATTCATCATCGAAGTAC |
| 2. | | | V2LHS_132502 | TGACAAAGGTGTTGGGTCG |
| 3. | | | V3LHS_636396 | GTGTGAGCGACTTCATCCT |
| 4. | | | V3LHS_646518 | TGATGCTGAGGAAGAACCT |
| 5. | | | V3LHS_636398 | CATCATCGAAGTACCTTGT |
| 6. | | | V3LHS_636400 | TTGATGACAGACACCTCAT |
| 7. | | | V3LHS_325557 | TCTTTGATGACAGACACCT |
| 8. | ALDH1A1 | Retinal dehydrogenase 1 P00352 | V2LHS_112035 | TTATTAAAGATGCCACGTG |
| 9. | | | V2LHS_265598 | AAAGACAGGAAATTTCTTG |
| 10. | | | V2LHS_112039 | ATGTCTTTGGTAAACACTC |
| 11. | | | V2LHS_112037 | ATCCATGTGAGAAGAAATG |
| 12. | | | V3LHS_398453 | ACTTTGTCTATATCCATGT |
| 13. | | | V3LHS_398455 | AATTCAACAGCATTGTCCA |
| 14. | AURKB | Aurora kinase B Q96GD4 | V2LHS_ 28602 | TAAGGGAACAGTTAGGGAT |
| 15. | | | V2LHS_28606 | ATGACAGGGACCATCAGGC |
| 16. | | | V2LHS_28601 | TTCTCCATCACCTTCTGGC |
| 17. | | | V3LHS_341839 | TCAAGTAGATCCTCCTCCG |
| 18. | | | V3LHS_341836 | ATGTCTCTGTGAATCACCT |
| 19. | | | V3LHS_341841 | TCGATCTCTCTGCGCAGCT |
| 20. | | | V3LHS_341840 | AGAGCATCTGCCAACTCCT |
| 21. | | | V3LHS_341837 | TTTCTGGCTTTATGTCTCT |
| 22. | BCAM | Basal Cell Adhesion Molecule P50895 | V2LHS_62437 | ATAATGGTCGTGGGTTCCC |
| 23. | | | V2LHS_62435 | TTGCAAACACGTTGAGCCG |
| 24. | | | V3LHS_323253 | AATCCTCCACTCTGCAGCC |
| 25. | | | V3LHS_323254 | TCCGCTGTCTTTAGCTCTG |
| 26. | | | V3LHS_323256 | TGAGTGTGACTTCGTCTCC |
| 27. | | | V3LHS_323255 | GTGACTTCGTCTCCTTCCC |
| 28. | | | V3LHS_323251 | AGAGGTAAGGAAAGCACCT |
| 29. | BIRC5 | Baculoviral IAP repeat-containing protein 5 O15392 | V2LHS_94585 | ATCAAATCCATCATCTTAC |
| 30. | | | V2LHS_94582 | TAAACAGTAGAGGAGCCAG |
| 31. | | | V2LHS_262796 | AGCAGAAGAAACACTGGGC |
| 32. | | | V2LHS_262484 | TTCCTAAGACATTGCTAAG |
| 33. | | | V2LHS_230582 | TCTTGAATGTAGAGATGCG |
| 34. | | | V3LHS_350788 | AATTCTTCAAACTGCTTCT |
| 35. | | | V3LHS_350789 | TGTTCTTGGCTCTTTCTCT |
| 36. | | | V3LHS_383705 | TGAAGCAGAAGAAACACTG |
| 37. | | | V3LHS_383704 | GAAGCAGAAGAAACACTGG |
| 38. | CCNB1 | G2/mitotic-specific cyclin-B1 P14635 | V3LHS_369356 | TTACCATGACTACATTCTT |
| 39. | | | V3LHS_369358 | TGCTTGCAATAAACATGGC |
| 40. | | | V3LHS_369355 | TAATTTTCGAGTTCCTGGT |
| 41. | | | V3LHS_369360 | AAAGCTCTTAGAATCTTCA |
| 42. | | | V3LHS_369359 | AGAATCTTCATTTCCATCT |
| 43. | CD133 | Prominin-1 O43490 | V2LHS_71816 | ATCATTAAGGGATTGATAG |
| 44. | | | V2LHS_71820 | TTATACAAATCACCAACAG |
| 45. | | | V2LHS_ 71818 | TAGTAGACAATCTTTAGAC |
| 46. | | | V2LHS_71819 | TGTTCTATAGGAAGGACTC |
| 47. | | | V3LHS_407402 | TTCATTTTAGAACACTTGA |
| 48. | | | V3LHS_352745 | ATAGGAAGGACTCGTTGCT |
| 49. | | | V3LHS_352742 | ATAGTTTCAACATCATCGT |
| 50. | | | V3LHS_352743 | ATTATTATACAAATCACCA |
| 51. | CD44 | CD44 antigen, Receptor for hyaluronic acid (HA) P16070 | V2LHS_111680 | TATATTCAAATCGATCTGC |
| 52. | | | V2LHS_111682 | ATATGTGTCATACTGGGAG |
| 53. | | | V2LHS_111684 | AATGGTGTAGGTGTTACAC |
| 54. | | | V3LHS_334831 | AGAGTTGGAATCTCCAACA |
| 55. | | | V3LHS_334830 | TGGGTCTCTTCTTCCACCT |
| 56. | | | V3LHS_334834 | TGTGCTTGTAGAATGTGGG |
| 57. | | | V3LHS_334832 | TGTCTGAAGTAGCACTTCC |
| 58. | CDC20 | Cell division cycle protein 20 homolog Q12834 | V2LHS_112883 | TTCCAGATGCGAATGTGTC |
| 59. | | | V2LHS_112884 | ATAACTAGCTGGTTCTGTG |
| 60. | | | V3LHS_640507 | AACTAGCTGGTTCTGTGCA |
| 61. | | | V3LHS_640508 | CAGGTAATAGTCATTTCGG |
| 62. | | | V3LHS_645717 | AAACAACTGAGGTGATGGG |
| 63. | | | V3LHS_645716 | AATAAAAAACAACTGAGGT |
| 64. | | | V3LHS_640514 | ACTTCCAAATAACTAGCTG |
| 65. | | | V3LHS_363298 | TCTGCTGCTGCACATCCCA |
| 66. | CDKN2D | Cyclin-dependent kinase 4 inhibitor D P55273 | V2LHS_262156 | AATAAATAGAATCCATTTC |
| 67. | | | V3LHS_ 401207 | ATGAATAACTCATAACTCA |
| 68. | | | V3LHS_310385 | CCACTAGGACCTTCAGGGT |
| 69. | | | V3LHS_310386 | CGGGATGCACCAGCTCGCG |
| 70. | | | V3LHS_310389 | AGGACCTTCAGGGTGTCCA |
| 71. | | | V3LHS_310387 | GAACTGCCAGATGGATTGG |
| 72. | CDKN3 | Cyclin-dependent kinase inhibitor 3 Q16667 | V2LHS_262397 | TATAGTAGGAGACAAGCAG |
| 73. | | | V2LHS_201585 | TGCTTGATGGTCTGTATTG |
| 74. | | | V3LHS_386043 | TGATTGTGAATCTCTTGAT |
| 75. | | | V3LHS_386040 | ATCTTGATACAGATCTTGA |
| 76. | | | V3LHS_386041 | TGATACAGATCTTGATTGT |
| 77. | CENPA | Histone H3-like centromeric protein A P49450 | V2LHS_150535 | ATATGATGGAAATGCCCAG |
| 78. | | | V2LHS_150534 | TATTACCTCTGTTACAGAG |
| 79. | | | V2LHS_150531 | TAACACATATTTCTCTTGC |
| 80. | | | V3LHS_403419 | AAAGCAACACACACATACT |
| 81. | | | V3LHS_403420 | AGACTGACAGAAACACTGG |
| 82. | | | V3LHS_403421 | TGTCTCATATATTACCTCT |
| 83. | | | V3LHS_403422 | TATCTGAAAATTATTTTCA |
| 84. | | | V3LHS_313522 | TTGGGAAGAGAGTAACTCG |
| 85. | CIP2A | CIP2A (gene KIAA1524) Q8TCG1 | V2LH_206422 | TACTCAATGTCTTTATGTG |
| 86. | | | V3LHS_308568 | TGAATGTGATCTATCAGGA |
| 87. | | | V3LHS_308569 | TGTTCTCTATTATCTGACG |
| 88. | | | V3LHS_308565 | TTCATTTCATATACATCCA |
| 89. | | | V3LHS_308566 | TGAACAGAAAGATTGTGCC |
| 90. | FOXM1 | Forkhead box protein M1 Q08050 | V2LHS_283849 | ATAATTAGAGGATAATTTG |
| 91. | | | V3LHS_396939 | ATTGTTGATAGTGCAGCCT |
| 92. | | | V3LHS_396937 | TGAATCACAAGCATTTCCG |
| 93. | | | V3LHS_396941 | TGATGGTCATGTTCCGGCG |
| 94. | | | V3LHS_396940 | AATAATCTTGATCCCAGCT |
| 95. | PLK1 | Serine/threonine-protein kinase PLK1 P53350 | V2LHS_19709 | ATTCTGTACAATTCATATG |
| 96. | | | V2LHS_19711 | ATAGCCAGAAGTAAAGAAC |
| 97. | | | V2LHS_241437 | TGCGGAAATATTTAAGGAG |
| 98. | | | V2LHS_19708 | GTAATTAGGAGTCCCACAC |
| 99. | | | V2LHS_262328 | AATTAGGAGTCCCACACAG |
| 100 | | | V3LHS_311459 | TTCTTGCTCAGCACCTCGG |
| 101 | | | V3LHS_311462 | TTGACACTGTGCAGCTGCT |
| 102 | | | V3LHS_311463 | TAGGCACAATCTTGCCCGC |
| 103 | PRDM16 | PR domain zinc finger protein 16 Q9HAZ2 | V2LHS_215636 | TAAAGCCTCAGAATCTAAG |
| 104 | | | V2LHS_251390 | TAAATTACGACTCTGACAC |
| 105 | | | V3LHS_300082 | ATTATTTACAACGTCACCG |
| 106 | | | V3LHS_300078 | TTCTCGTCTAAAAGTGCGT |
| 107 | | | V3LHS_300081 | AAAAGTGCGTGGTTGTCCG |
| 108 | SALL4 | Sal-like protein 4 Q9UJQ4 | V3LHS_363661 | TAGCTGACCGCAATCTTGT |
| 109 | | | V3LHS_363659 | TAGTGAACTTCTTCTGGCA |
| 110 | | | V3LHS_363662 | TCGGCTTGACTATTGGCCG |
| 111 | | | V3LHS_363664 | TTCTGAGACTCTTTTTCCG |
| 112 | SLC25A6 | ADP/ATP translocase 3 (gene SLC25A6) P12236 | V3LHS_314256 | TGTACTTATCCTTGAAGGC |
| 113 | | | V3LHS_314257 | TGCCCGCAAAGTACCTCCA |
| 114 | WDFY2 | WD repeat and FYVE domain-containing protein 2 Q96P53 | V2LHS_118254 | TATCCCACAACTTAATAAC |
| 115 | | | V2LHS_118249 | TAACCAAACACGAACTGTC |
| 116 | | | V3LHS_405758 | ATTGTATGAACAAGTTGGA |
| 117 | | | V3LHS_341295 | TTCACAGGAGTCATCTTGT |
| 118 | | | V3LHS_405756 | TATATTGTATGAACAAGTT |
| 119 | CBCP1 | cyclin Y | V2LHS_243158 | ATACTTGGCATAGACACTG |
| 120 | | | V3LHS_314369 | TACTGAGGAATATTGTGCT |
| 121 | | | V3LHS_314371 | TAATGAAGAGACTCTTGCG |

**Table 2. MiniVector elements**

| **Table 2.** MiniVector elements | | | |
|---|---|---|---|
| Module | Element | Description | Use |
| A | λ-*att*L | *att*L from the λ-integrase system | Recombination sites (product of site-specific recombination used to generate MiniVector). Sequences listed in Table 3. |
| | λ-*att*R | *att*R from the λ-integrase system | |
| | λ-*att*B | *att*B from the λ-integrase system | |
| | λ-*att*P | *att*P from the λ-integrase system | |
| | *lox*P | *lox*P site for Cre recombinase | |
| | γδ-*res* | *res* site for the yδ (Tn1000) resolvase | |
| | FRT | FRT site for Flp recombinase | |
| | *hixL* | *hix*L site for Hin recombinase | |
| | *hixR* | *hix*R site for Hin recombinase | |
| | Tn3 *res* | *res* site for Tn3 resolvase | |
| | Tn21 *res* | *res* site for Tn21 resolvase | |
| | *cer* | cer site for XerCD system | |
| | *psi* | *psi* site for XerCD | |
| B | | Tissue-specific promoter of alcohol dehydrogenase 1 (ALDH1) | Initiation of transcription. Includes promoters for RNA polymerase II and RNA polymerase III. Full sequences of selected promoters provided in Table 4. |
| | AMY1C | Tissue-specific promoter of human amylase alpha 1C (AMY1C) | |
| | β-actin | Promoter from the (human) beta actin gene | |
| | CaMKIIα | Ca2+/calmodulin-dependent protein kinase II alpha promoter | |
| | CMV | Promoter from the human cytomegalovirus (CMV) | |
| | Mini CMV | Minimized version of CMV | |
| | CAG | CMV early enhancer/chicken β actin promoter (CAG). Synthetic hybrid promoter made from a) the CMV early enhancer element, b) the promoter, the first exon and the first intron of chicken beta-actin gene, and c) the splice acceptor of the rabbit beta-globin gene | |
| | Cytokeratin 18 and 19 | Cell-specific promoters of the human keratin 18 and 19 genes | |
| | EF1α | Strong expression promoter from human elongation factor 1 alpha | |
| | GFAP | Tissue-specific promoter of the glial fibrillary acidic protein (GFAP) | |
| | | Promoter from the human polymerase III RNA promoter | |
| | Kallikrein | Tissue-specific promoter of the kallikrein gene. | |
| | NFK-β | Nuclear factor kappa-light-chain-enhancer of activated B cells (NF-Kβ) | |
| | PGK1 | Promoter from human or mouse phosphoglycerate kinase gene (PGK) | |
| | RSV | Long terminal repeat (LTR) of the rous sarcoma virus (RSV) | |
| | SV40 | Mammalian expression promoter from the simian vacuolating virus 40 | |
| | UBC | Promoter of the human ubiquitin C gene (UBC) | |
| | U6 | Promoter from the human U6 small nuclear promoter | |
| C | shRNA | (DNA) sequence encoding short hairpin RNA (shRNA) transcript. Sequences for use in target validation are listed in Table 1. Potential therapeutic sequences will be designed *de novo* and optimized for knockdown efficiency. | Knockdown of gene expression through RNA interference |
| | miRNA | (DNA) sequence encoding micro-RNA (miRNA) transcript | |
| | IhRNA | (DNA) sequence encoding long hairpin RNA (lhRNA) transcript | |
| | lncRNA | (DNA) sequence encoding long non-coding RNA (IncRNA) transcript | Knockdown of gene expression (not RNAi) |
| | piRNA | (DNA) sequence encoding piwi-interacting (piRNA) RNA transcript | |
| D | | Transcriptional terminator sequence | |
| E | S/MAR | Scaffold/matrix attached region from eukaryotic chromosomes (Sequences in Table 5) | Episomal replication |
| | CpG motifs | Unmethylated deoxycytidyl-deoxyguanosine (CpG) dinucleotides: (Sequences in Table 5) | Immunostimulato ry activity |
| F/G | β-globin intron | Intron of the human β globin gene (130 bp) | Gene expression enhancer |
| | HGH intron | Intron of the human growth hormone gene (262 bp) | |
| H | SV40 early promoter | Simian virus 40 early promoter (351 bp) | Nuclear localization |
| | NF-κβ | Binding site of nuclear factor kappa-light-chain-enhancer of activated B cells (55 bp (5 repeats of GGGGACTTTCC SEQ ID NO 122)) | |
| | p53NLS | Binding site of tumor protein 53 (p53): AGACTGGGCATGTCTGGGCA SEQ ID NO 123 | |
| | p53 NLS | Binding site of tumor protein 53 (p53): GAACATGTCCCAACATGTTG SEQ ID NO 124 | |
| | Adenovirus major late promoter | GGGGCTATAAAAGGG SEQ ID NO 125 | |

**Table 3. Complete sequences for element A (recombination sites)**

| SEQ ID NO | Site | Sequence (5'-3') |
|---|---|---|
| 126. | λ-*att*L | TCCGTTGAAGCCTGCTTTTTTATACTAAGTTGGCATTATAAAAAAGCATTGC TTATCAATTTGTTGCAACGAACAGGTCACTATCAGTCAAAATAAAATCATTA TT |
| 127. | λ-*att*R | AGATGCCTCAGCTCTGTTACAGGTCACTAATACCATCTAAGTAGTTGATTCA TAGTGACTGCATATGTTGTGTTTTACAGTATTATGTAGTCTGTTTTTTATGC AAAATCTAATTTAATATATTGATATTTATATCATTTTACGTTTCTCGTTCAG CTTTTTTATACTAACTTGAGCGAAACG |
| 128. | λ-*att*B | TCCGTTGAAGCCTGCTTTTTTATACTAACTTGAGCGAAACG |
| 129. | λ-*att*P | AGATGCCTCAGCTCTGTTACAGGTCACTAATACCATCTAAGTAGTTGATTCA TAGTGACTGCATATGTTGTGTTTACAGTATTATGTAGTCTGTTTTTATGC AAAATCTAATTTAATATATTGATATTTATATCATTTTACGTTTCTCGTTCAG CTTTTTTATACTAAGTTGGCATTATAAAAAGCATTGCTTATCAATTTGTTG CAACGAACAGGTCACTATCAGTCAAAATAAAATCATTATT |
| *130.* | *lox*P | ATAACTTCGTATAGCATACATTATACGAAGTTAT |
| 131. | γδ-*res* | ATTTTGCAACCGTCCGAAATATTATAAATTATCGCACACATAAAAACAGTGC TGTTAATGTGTCTATTAAATCGATTTTTTGTTATAACAGACACTGCTTGTCC GATATTTGATTTAGGATACATTTTTA |
| 132. | FRT | GAAGTTCCTATTCTCTAGAAAGTATAGGAACTTC |
| *133.* | *hix*L | TTCTTGAAAACCAAGGTTTTTGATAA |
| 134. | *hix*R | TTTTCCTTTTGGAAGGTTTTTGATAA |
| 135. | Tn3 *res* | CAACCGTTCGAAATATTATAAATTATCAGACATAGTAAAACGGCTTCGTTTG AGTGTCCATTAAATCGTCATTTTGGCATAATAGACACATCGTGTCTGATATT CGATTTAAGGTACATTT |
| 136. | Tn21 *res* | GCCGCCGTCAGGTTGAGGCATACCCTAACCTGATGTCAGATGCCATGTGTAA ATTGCGTCAGGATAGGATTGAATTTTGAATTTATTGACATATCTCGTTGAAG GTCATAGAGTCTTCCCTGACAT |
| 137. | | GGTGCGTACAATTAAGGGATTATGGTAAAT |
| 138. | | GGTGCGCGCAAGATCCATTATGTTAAAC |

**Table 4. Complete sequences for element B (promoters)**

| SEQ ID NO | Promoter | Sequence (5'-3') |
|---|---|---|
| 139. | CMV | GACATTGATTATTGACTAGTTATTAATAGTAATCAATTACGGGGTCATTAGT TCATAGCCCATATATGGAGTTCCGCGTTACATAACTTACGGTAAATGGCCCG CCTGGCTGACCGCCCAACGACCCCCGCCCATTGACGTCAATAATGACGTATG TTCCCATAGTAACGCCAATAGGGACTTTCCATTGACGTCAATGGGTGGAGTA TTTACGGTAAACTGCCCACTTGGCAGTACATCAAGTGTATCATATGCCAAGT ACGCCCCCTATTGACGTCAATGACGGTAAATGGCCCGCCTGGCATTATGCCC AGTACATGACCTTATGGGACTTTCCTACTTGGCAGTACATCTACGTATTAGT CATCGCTATTACCATGGTGATGCGGTTTTGGCAGTACATCAATGGGCGTGGA TAGCGGTTTGACTCACGGGGATTTCCAAGTCTCCACCCCATTGACGTCAATG GGAGTTTGTTTTGGCACCAAAATCAACGGGACTTTCCAAAATGTCGTAACAA CTCCGCCCCATTGACGCAAATGGGCGGTAGGCGTGTACGGTGGGAGGTCTAT ATAAGCAGAGCT |
| 140. | mini-CMV | CCAAAATCAACGGGACTTTCCAAAATGTCGTAACAACTCCGCCCCATTGACG CAAATGGGCGGTAGGCGTGTACGGTGGGAGGTCTATATAAGCAGAGCT |
| 141. | RSV | GGTGCACACCAATGTGGTGAATGGTCAAATGGCGTTTATTGTATCGAGCTAG GCACTTAAATACAATATCTCTGCAATGCGGAATTCAGTGGTTCGTCCAATCC ATGTCAGACCCGTCTGTTGCCTTCCTAATAAGGCACGATCGTACCACCTTAC TTCCACCAATCGGCATGCACGGTGCTTTTTCTCTCCTTGTAAGGCATGTTGC TAACTCATCGTTACCATGTTGCAAGACTACAAGAGTATTGCATAAGACTACA TT |
| 142. | CAG | GCGTTACATAACTTACGGTAAATGGCCCGCCTGGCTGACCGCCCAACGACCC CCGCCCATTGACGTCAATAATGACGTATGTTCCCATAGTAACGCCAATAGGG ACTTTCCATTGACGTCAATGGGTGGAGTATTTACGGTAAACTGCCCACTTGG CAGTACATCAAGTGTATCATATGCCAAGTACGCCCCCTATTGACGTCAATGA CGGTAAATGGCCCGCCTGGCATTATGCCCAGTACATGACCTTATGGACTTT CCTACTTGGCAGTACATCTACGTATTAGTCATCGCTATTACCATGGTCGAGG TGAGCCCCACGTTCTGCTTCACTCTCCCCATCTCCCCCCCCTCCCCACCCCC AATTTTGTATTTATTTATTTTTTAATTATTTTGTGCAGCGATGGGGGCGGGG GGGGGGGGGGGCGCGCGCCAGGCGGGGCGGGGCGGGGCGAGGGGCGGGGCG GGGCGAGGCGGAGAGGTGCGGCGGCAGCCAATCAGAGCGGCGCGCTCCGAAA GTTTCCTTTTATGGCGAGGCGGCGGCGGCGGCGGCCCTATAAAAAGCGAAGC GCGCGGCGGGCG |
| 143. | EF1a | GCTCCGGTGCCCGTCAGTGGGCAGAGCGCACATCGCCCACAGTCCCCGAGAA GTTGGGGGGAGGGGTCGGCAATTGAACCGGTGCCTAGAGAAGGTGGCGCGGG GTAAACTGGGAAAGTGATGTCGTGTACTGGCTCCGCCTTTTTCCCGAGGGTG GGGGAGAACCGTATATAAGTGCAGTAGTCGCCGTGAACGTTCTTTTTCGCAA CGGGTTTGCCGCCAGAACACAGGTAAGTGCCGTGTGTGGTTCCCGCGGGCCT GGCCTCTTTACGGGTTATGGCCCTTGCGTGCCTTGAATTACTTCCACGCCCC TGGCTGCAGTACGTGATTCTTGATCCCGAGCTTCGGGTTGGAAGTGGGTGGG AGAGTTCGAGGCCTTGCGCTTAAGGAGCCCCTTCGCCTCGTGCTTGAGTTGA GGCCTGGCCTGGGCGCTGGGGCCGCCGCGTGCGAATCTGGTGGCACCTTCGC GCCTGTCTCGCTGCTTTCGATAAGTCTCTAGCCATTTAAAATTTTTGATGAC CTGCTGCGACGCTTTTTTTCTGGCAAGATAGTCTTGTAAATGCGGGCCAAGA TCTGCACACTGGTATTTCGGTTTTTGGGGCCGCGGGCGGCGACGGGGCCCGT GCGTCCCAGCGCACATGTTCGGCGAGGCGGGGCCTGCGAGCGCGGCCACCGA GAATCGGACGGGGGTAGTCTCAAGCTGGCCGGCCTGCTCTGGTGCCTGGCCT CGCGCCGCCGTGTATCGCCCCGCCCTGGGCGGCAAGGCTGGCCCGGTCGGCA CCAGTTGCGTGAGCGGAAAGATGGCCGCTTCCCGGCCCTGCTGCAGGGAGCT CAAAATGGAGGACGCGGCGCTCGGGAGAGCGGGCGGGTGAGTCACCCACACA |
| | | AAGGAAAAGGGCCTTTCCGTCCTCAGCCGTCGCTTCATGTGACTCCACGGAG TACCGGGCGCCGTCCAGGCACCTCGATTAGTTCTCGAGCTTTTGGAGTACGT CGTCTTTAGGTTGGGGGGAGGGGTTTTATGCGATGGAGTTTCCCCACACTGA GTGGGTGGAGACTGAAGTTAGGCCAGCTTGGCACTTGATGTAATTCTCCTTG GAATTTGCCCTTTTTGAGTTTGGATCTTGGTTCATTCTCAAGCCTCAGACAG TGGTTCAAAGTTTTTTTCTTCCATTTCAGGTGTCGTGA |
| 144. | EFS | ATCGATTGGCTCCGGTGCCCGTCAGTGGGCAGAGCGCACATCGCCCACAGTC CCCGAGAAGTTGGGGGGAGGGGTCGGCAATTGAACCGGTGCCTAGAGAAGGT GGCGCGGGGTAAACTGGGAAAGTGATGTCGTGTACTGGCTCCGCCTTTTTCC CGAGGGTGGGGGAGAACCGTATATAAGTGCAGTAGTCGCCGTGAACGTTCTT TTTCGCAACGGGTTTGCCGCCAGAACACAGGTGTCGTGACGCG |
| 145. | Human β-actin | GGCCTCCGCGCCGGGTTTTGGCGCCTCCCGCGGGCGCCCCCCTCCTCACGGC GAGCGCTGCCACGTCAGACGAAGGGCGCAGCGAGCGTCCTGATCCTTCCGCC CGGACGCTCAGGACAGCGGCCCGCTGCTCATAAGACTCGGCCTTAGAACCCC AGTATCAGCAGAAGGACATTTTAGGACGGGACTTGGGTGACTCTAGGGCACT GGTTTTCTTTCCAGAGAGCGGAACAGGCGAGGAAAAGTAGTCCCTTCTCGGC GATTCTGCGGAGGGATCTCCGTGGGGCGGTGAACGCCGATGATTATATAAGG ACGCGCCGGGTGTGGCACAGCTAGTTCCGTCGCAGCCGGGATTTGGGTCGCG GTTCTTGTTTGTGGATCGCTGTGATCGTCACTTGGTGAGTAGCGGGCTGCTG GGCTGGCCGGGGCTTTCGTGGCCGCCGGGCCGCTCGGTGGGACGGAAGCGTG TGGAGAGACCGCCAAGGGCTGTAGTCTGGGTCCGCGAGCAAGGTTGCCCTGA ACTGGGGGTTGGGGGGAGCGCAGCAAAATGGCGGCTGTTCCCGAGTCTTGAA TGGAAGACGCTTGTGAGGCGGGCTGTGAGGTCGTTGAAACAAGGTGGGGGGC ATGGTGGGCGGCAAGAACCCAAGGTCTTGAGGCCTTCGCTAATGCGGGAAAG CTCTTATTCGGGTGAGATGGGCTGGGGCACCATCTGGGGACCCTGACGTGAA GTTTGTCACTGACTGGAGAACTCGGTTTGTCGTCTGTTGCGGGGGCGGCAGT TATGGCGGTGCCGTTGGGCAGTGCACCCGTACCTTTGGGAGCGCGCGCCCTC GTCGTGTCGTGACGTCACCCGTTCTGTTGGCTTATAATGCAGGGTGGGGCCA CCTGCCGGTAGGTGTGCGGTAGGCTTTTCTCCGTCGCAGGACGCAGGGTTCG GGCCTAGGGTAGGCTCTCCTGAATCGACAGGCGCCGGACCTCTGGTGAGGGG AGGGATAAGTGAGGCGTCAGTTTCTTTGGTCGGTTTTATGTACCTATCTTCT TAAGTAGCTGAAGCTCCGGTTTTGAACTATGCGCTCGGGGTTGGCGAGTGTG TTTTGTGAAGTTTTTTAGGCACCTTTTGAAATGTAATCATTTGGGTCAATAT GTAATTTTCAGTGTTAGACTAGTAAATTGTCCGCTAAATTCTGGCCGTTTTT GGCTTTTTTGTTAGAC |
| 146. | NFK-β | GCTAGCGGGAATTTCCGGGAATTTCCGGGAATTTCCGGGAATTTCCAGATCT GCCGCCCCGACTGCATCTGCGTGTTCGAATTCGCCAATGACAAGACGCTGGG CGGGGTTTGTGTCATCATAGAACTAAAGACATGCAAATATATTTCTTCCGGG GACACCGCCAGCAAACGCGAGCAACGGGCCACGGGGATGAAGCAGAAGCTTG GCA |
| 147. | Ubiquitin-C | GTCTAACAAAAAGCCAAAAACGGCCAGAATTTAGCGGACAATTTACTAGTC TAACACTGAAAATTACATATTGACCCAAATGATTACATTTCAAAAGGTGCCT AAAAAACTTCACAAAACACACTCGCCAACCCCGAGCGCATAGTTCAAAACCG GAGCTTCAGCTACTTAAGAAGATAGGTACATAAAACCGACCAAAGAAACTGA CGCCTCACTTATCCCTCCCCTCACCAGAGGTCCGGCGCCTGTCGATTCAGGA GAGCCTACCCTAGGCCCGAACCCTGCGTCCTGCGACGGAGAAAAGCCTACCG CACACCTACCGGCAGGTGGCCCCACCCTGCATTATAAGCCAACAGAACGGGT GACGTCACGACACGACGAGGGCGCGCTCCCAAAGGTACGGGTGCACTGCC CAACGGCACCGCCATAACTGCCGCCCCCGCAACAGACGACAAACCGAGTTCT CCAGTCAGTGACAAACTTCACGTCAGGGTCCCCAGATGGTGCCCCAGCCCAT CTCACCCGAATAAGAGCTTTCCCGCATTAGCGAAGGCCTCAAGACCTTGGGT |
| | | TCTTGCCGCCCACCATGCCCCCCACCTTGTTTCAACGACCTCACAGCCCGCC TCACAAGCGTCTTCCATTCAAGACTCGGGAACAGCCGCCATTTTGCTGCGCT CCCCCCAACCCCCAGTTCAGGGCAACCTTGCTCGCGGACCCAGACTACAGCC CTTGGCGGTCTCTCCACACGCTTCCGTCCCACCGAGCGGCCCGGCGGCCACG AAAGCCCCGGCCAGCCCAGCAGCCCGCTACTCACCAAGTGACGATCACAGCG ATCCACAAACAAGAACCGCGACCCAAATCCCGGCTGCGACGGAACTAGCTGT GCCACACCCGGCGCGTCCTTATATAATCATCGGCGTTCACCGCCCCACGGAG ATCCCTCCGCAGAATCGCCGAGAAGGGACTACTTTTCCTCGCCTGTTCCGCT CTCTGGAAAGAAAACCAGTGCCCTAGAGTCACCCAAGTCCCGTCCTAAAATG TCCTTCTGCTGATACTGGGGTTCTAAGGCCGAGTCTTATGAGCAGCGGGCCG CTGTCCTGAGCGTCCGGGCGGAAGGATCAGGACGCTCGCTGCGCCCTTCGTC TGACGTGGCAGCGCTCGCCGTGAGGAGGGGGGCGCCCGCGGGAGGCGCCAAA ACCCGGCGCGGAGGC |
| 148. | SV40 | GGTGTGGAAAGTCCCCAGGCTCCCCAGCAGGCAGAAGTATGCAAAGCATGCA TCTCAATTAGTCAGCAACCAGGTGTGGAAAGTCCCCAGGCTCCCCAGCAGGC AGAAGTATGCAAAGCATGCATCTCAATTAGTCAGCAACCATAGTCCCGCCCC TAACTCCGCCCATCCCGCCCCTAACTCCGCCCAGTTCCGCCCATTCTCCGCC CCATGGCTGACTAATTTTTTTTATTTATGCAGAGGCCGAGGCCGCCTCGGCC TCTGAGCTATTCCAGAAGTAGTGAGGAGGCTTTTTTGGAGGCCTAGGCTTTT GCAAA |
| 149. | PGK | CCGGTAGGCGCCAACCGGCTCCGTTCTTTGGTGGCCCCTTCGCGCCACCTTC TACTCCTCCCCTAGTCAGGAAGTTCCCCCCCGCCCCGCAGCTCGCGTCGTGC AGGACGTGACAAATGGAAGTAGCACGTCTCACTAGTCTCGTGCAGATGGACA GCACCGCTGAGCAATGGAAGCGGGTAGGCCTTTGGGGCAGCGGCCAATAGCA GCTTTGCTCCTTCGCTTTCTGGGCTCAGAGGCTGGGAAGGGGTGGGTCCGGG GGCGGGCTCAGGGGCGGGCTCAGGGGCGGGGCGGGCGCCCGAAGGTCCTCCG GAGGCCCGGCATTCTGCACGCTTCAAAAGCGCACGTCTGCCGCGCTGTTCTC CTCTTCCTCATCTCCGGGCCTTTCGACCTGCAGCC |
| 150. | H1 | AATATTTGCATGTCGCTATGTGTTCTGGGAAATCACCATAAACGTGAAATGT CTTTGGATTTGGGAATCTTATAAGTTCTGTATGAGACCACAGATCCC |
| 151. | U6 | GATCCGACGCCGCCATCTCTAGGCCCGCGCCGGCCCCCTCGCACAGACTTGT GGGAGAAGCTCGGCTACTCCCCTGCCCCGGTTAATTTGCATATAATATTTCC TAGTAACTATAGAGGCTTAATGTGCGATAAAAGACAGATAATCTGTTCTTTT TAATACTAGCTACATTTTACATGATAGGCTTGGATTTCTATAAGAGATACAA ATACTAAATTATTATTTTAAAAAACAGCACAAAAGGAAACTCACCCTAACTG TAAAGTAATTGTGTGTTTTGAGACTATAAATATCCCTTGGAGAAAAGCCTTG TT |

**Table 5. Complete sequences for elements E, F and G (accessory sequences)**

| SEQ ID NO | Element | Sequence (5'-3') |
|---|---|---|
| 152. | 250 bp S/MAR | TCTTTAATTTCTAATATATTTAGAATCTTTAATTTCTAATATATTTAG AATCTTTAATTTCTAATATATTTAGAATCTTTAATTTCTAATATATTT AGAATCTTTAATTTCTAATATATTTAGAATCTTTAATTTCTAATATAT TTAGAATCTTTAATTTCTAATATATTTAGAATCTTTAATTTCTAATAT ATTTAGAATCTTTAATTTCTAATATATTTAGAATCTTTAATTTCTAAT ATATTTAGAA |
| 153. | 439 bp S/MAR | TCTTTAATTTCTAATATATTTAGAATCTTTAATTTCTAATATATTTAG AATCTTTAATTTCTAATATATTTAGAATCTTTAATTTCTAATATATTT AGAATCTTTAATTTCTAATATATTTAGAATCTTTAATTTCTAATATAT TTAGAATCTTTAATTTCTAATATATTTAGAATCTTTAATTTCTAATAT ATTTAGAATCTTTAATTTCTAATATATTTAGAATCTTTAATTTCTAAT ATATTTAGAA |
| 154. | (45 bp) Type A Cpg motif | GGTGCATCGATGCAGCATCGAGGCAGGTGCATCGATACAGGGGGG |
| 155. | (24 bp) Type B Cpg motif | TCGTCGTTTTGTCGTTTTGTCGTT |
| 156. | (21 bp) Type C CpG motif | TCGTCGAACGTTCGAGATGAT |
| 157. | β-globin intron | GTTGGTATCAAGGTTACAAGACAGGTTTAAGGAGACCAATAGAAACTG GGCATGTGGAGACAGAGAAGACTCTTGGGTTTCTGATAGGCACTGACT CTCTCTGCCTATTGGTCTATTTTCCCACCCTTAG |
| 158. | Human growth hormone intron | TTCGAACAGGTAAGCGCCCCTAAAATCCCTTTGGGCACAATGTGTCCT GAGGGGAGAGGCAGCGACCTGTAGATGGACGGGGGCACTAACCCTCA GGTTTGGGGCTTCTGAATGTGAGTATCGCCATGTAAGCCCAGTATTTG GCCAATCTCAGAAAGCTCCTGGTCCCTGGAGGGATGGAGAGAGAAAAA CAAACAGCTCCTGGAGCAGGGAGAGTGCTGGCCTCTTGCTCTCCGGCT CCCTCTGTTGCCCTCTGGTTTC |

| **Table 6:** cancers and suitable test cell lines |
|---|
| Breast cancer: MDA-MB 231, SKBR3, SUM 102, SUM 149, and MCF-7. |
| Cervical cancer: Nos NC104, NC105, HeLa, SiHa, CasKi, C33A, and C4-1. |
| Colorectal cancer: LoVo, SW480, and SW116. |
| Stomach cancer: NCI-N87, AGS, HTB 103, HTB 135, SNU1, SNU16, Sk-GT5, BGC-823, HGC-27, and KATO-III. |
| Glioma: Hs683, U118MG, LN 229, U87 MG, HF-U251 MG, SW1783, and U87 MG. |
| Pancreatic cancer: AsPC-1, BxPC-3, COLO-357, HPAC, L3.6PI, MIAPaCa, PANC1, CaPan-1, MiaPaca-2, MDAPanc-28, and MDAPanc-48. |
| Prostate cancer: PC-3, DU145, C4-2B, and LNCaP. |
| Kidney/renal cancer: 786-O and Caki-1. |
| Liver cancer: SK-Hep1, MHCC-LM3, and SMMC-7721. |
| Lung cancer: A549 and H1299. |
| Ovarian cancer: OVCAR8 and OV90. |
| Prostate cancer: PC3, DU145, and LNCaP. |
| Chondrasarcoma: HS-819.T, SW1353. |
| Dermatofibrosarcoma: Hs295.Sk, Hs357.T, Hs63.T. |
| Ewing sarcoma: SK-N-MC, TC-268, CHP-100S, CHP-100L, IMR-32, SK-ES1, ES4, WE68, RD-ES, HS863.T, HS822.T. |
| Fibrosarcoma: FC83.Res. |
| Giant cell sarcoma: Hs706.T, Hs737.T, Hs821.T, Hs127.T. |
| Leiomyosarcoma: HS5T, SK-LMS-1, DDT1 MF-2. |
| Liposarcoma: SW872. |
| Lung sarcoma: Hs57.T, LL86 |
| Lymphosarcoma: LB9.Bm, BL3.1. |
| Osteosarcoma: SK-ES-1. |
| Pagetoid sarcoma: Hs 925.T. |
| Reticulum cell sarcoma: J774A.1, HS324.T. |
| Rhabdomyosarcoma: TE381.T, TE441.T, RD, A-673, HS729, A-204, SJCRH30, TE159.T. |
| Uterine sarcoma: MES-SA, MES-SA/DX-5, MES-SA/MX2. |
| Lung sarcoma: HS-57.T, MiCl1 (S+L) |
| Other Sarcomas: FB2.K, DoCl1, CV-1, QNR/D, QNR/K2, 10.014 pRSV-T, 2.040pRSV-T, XMMCO-791, Hs925.Sk, Hs707(B).Ep, Sarcoma 180, EHS. |

| **Table 7:** Other Gene Therapy Targets | |
|---|---|
| Target | Type of cancer |
| AKAP12 | Glioma |
| AKT | Sarcomas |
| AKT1 | Lung cancer |
| ALK | Glioma Lung cancer |
| AR | Prostate Cancer |
| ARID1A | Cervical Cancer, Liver cancer |
| ARID2 | Liver cancer |
| AXIN1 | Liver cancer |
| AXL | Kidney/Renal Cancer |
| BARD1 | Glioma |
| BASP1 | Stomach Cancer |
| BCAR4 | Cervical, Colorectal, Stomach Cancer |
| Bcl-2 | Prostate Cancer |
| BCL-9 | Liver cancer |
| BIRC5 (Survivin) | Cervical Cancer |
| BLM | Glioma |
| Bmi1 | Stomach Cancer |
| BMP2 | Glioma |
| BRAF | Stomach. Lung cancer Glioma |
| BRCA1 | Breast Cancer, Glioma |
| BRCA2 | Breast Cancer |
| BRIP1 | Glioma |
| BUB1 | Glioma |
| c-met | Prostate Cancer |
| c-Myc | Stomach Cancer |
| CASP8 | Cervical Cancer |
| CAV1 | Kidney/Renal Cancer |
| CAV2 | Kidney/Renal Cancer |
| CBX7 | Stomach Cancer |
| CCNA2 | Glioma |
| CCND1 | Liver, Lung cancer |
| CCND2 | Glioma |
| CD274 (also known as PD-L1) | Cervical Cancer |
| CD44 | Stomach Cancer |
| CDC20 | Glioma |
| Cdc42 | Stomach Cancer |
| CDCA5 | Prostate Cancer |
| CDCA8 | Prostate Cancer |
| CDH1 | Stomach Cancer |
| CDK1 | Glioma |
| CDK2 | Glioma |
| CDK4 | Glioma |
| CDKN2A | Liver cancer |
| CDT-1 | Liver cancer |
| CDX1 | Stomach Cancer |
| CDX2 | Stomach Cancer |
| CEP55 | Glioma |
| CHEK1 | Glioma |
| COX-2 | Stomach Cancer |
| CTLA4 | Liver cancer |
| CTNNB1 aka Beta Catenin | Liver cancer |
| CyclinD2 | Stomach Cancer |
| DAB2IP | Glioma |
| DHX57-TMEM178-MAP4K3 | Glioma |
| DNMT | Liver cancer |
| DTMT1 | Stomach Cancer |
| DUSP26 | Glioma |
| E2F1 | Glioma |
| E2F2 | Prostate Cancer |
| ECOP | Stomach Cancer |
| EGF | Liver cancer |
| EGFR | Cervical, Colorectal, Lung Cancer, Glioma |
| EGFR-PSPH | Glioma |
| EGFR-SEPT14 | Glioma |
| EGR1 | Prostate Cancer |
| EML4-ALK | Lung cancer |
| EMSY | Breast Lung Cancer |
| EpCAM | Breast Cancer |
| ERBB3 | Cervical Cancer |
| ERG | Prostate Cancer |
| ERK | Liver cancer Sarcomas |
| Estrogen receptor (ER) | Breast cancer |
| FANCD2 | Glioma |
| Fat Specific Protein 27 aka DFFA-like effector aka CIDEC | Liver cancer |
| FGF19 | Liver cancer |
| FGF5 | Glioma |
| FGFR1 | Breast Cancer |
| FGFR1-4 | Liver cancer |
| FGFR1-TACC1 | Glioma |
| FGFR3-TACC3 | Glioma |
| FOXO1 | Stomach Cancer |
| G12C | Lung cancer |
| G12V | Lung cancer |
| GNAI1 | Kidney/Renal Cancer |
| GNAO1 | Kidney/Renal Cancer |
| GPSM2 | Kidney/Renal Cancer |
| GRPEL 1 | Liver cancer |
| HDAC | Liver cancer |
| HDAC | Sarcomas |
| HER-2 | Breast, Glioma, Liver, Lung cancer |
| HEYL | Glioma |
| HGESS | Sarcomas |
| HGF | Glioma |
| HIP1 | Prostate cancer |
| hK2 | Prostate Cancer |
| HLA-A | Cervical Cancer |
| HMGB2 | Glioma |
| HOXA1 | Cervical Cancer |
| ID4 | Glioma |
| IDH | Glioma, Sarcomas |
| IRF2 | Liver cancer |
| IRX5 | Glioma |
| ITGB3 | Glioma |
| JAK1 | Liver cancer |
| JAK2 | Stomach Cancer |
| KEAP1 | Liver cancer |
| KIT | Liver cancer |
| KRAS | Breast, Cervical, Colorectal, Stomach, Lung Cancer |
| LIMD1 | Glioma |
| LIN9 | Glioma |
| LKB1 | Cervical Cancer |
| LKB1 | Lung cancer |
| LPHN2 | Stomach Cancer |
| MAFG | Stomach Cancer |
| MAST1 | Ovarian cancer |
| Mcl-1 | Stomach Cancer |
| MDM2 | Sarcomas |
| MDM4 | Lung cancer |
| MeCP2 | Stomach Cancer |
| MEF2C | Glioma |
| MEIS2 | Glioma |
| MEK | Cervical, Liver Cancer, Glioma, Sarcomas |
| MEK1 | Lung cancer |
| MET | Glioma, Kidney/Renal Cancer, Liver cancer, Lung cancer |
| MET fusions: | Glioma |
| - TFG-MET | |
| - CLIP2-MET | |
| - PTPRZ1 (exon1)-MET | |
| - PTPRZ1 (exon2)-MET | |
| - PTPRZ1 (exon8)-MET | |
| MFSD2A | Glioma |
| mir-125b, mir-145, mir-21, and mir-155. | Breast Cancer |
| miR-203 | Cervical Cancer |
| miR-21, miR-17-5p, miR-191, miR-29b-2, miR-223, miR-128b, miR-24-1, miR-24-2, miR-155 | Colorectal Cancer |
| miR-21, miR-17-5p, miR-191, miR-29b-2, miR-223, miR-128b, miR-199a-1, miR-146, miR-181b-1 | Prostate Cancer |
| miR-21, miR-17-5p, miR-191, miR-29b-2, miR-223, miR-128b, miR-199a-1, miR-24-1, miR-24-2, miR-146, miR-181b-1 | Pancreatic Cancer |
| miR-21, miR-17-5p, miR-191, miR-128b, miR-199a-1, miR-155 | Lung cancer |
| miR-21, miR-17-5p, miR-29b-2, miR-146, miR-155, miR-181b-1 | Breast Cancer |
| miR-21, miR-191, miR-223, miR-24-1, miR-24-2 | Stomach Cancer |
| miR-221-3p | Cervical Cancer |
| miR-30b | Cervical Cancer |
| miR122 | Liver cancer |
| MN/CA9 | Cervical Cancer |
| mTOR | Kidney/Renal Cancer |
| mTOR, mTORC | Sarcomas |
| MYBL2 | Glioma |
| Myc | Prostate Cancer |
| NET1 | Glioma |
| NF1 | Glioma |
| NFE2L2 | Liver cancer |
| NOTCH4 | Stomach Cancer |
| NRAS | Lung cancer |
| NR3C4 (Androgen receptor) | Prostate cancer |
| NT53 | Glioma |
| NTRK fusions: | Glioma |
| - TPM3-NTRK1 | |
| - BTBD1-NTRK3 | |
| - ETV6-NTRK3 | |
| - VCL-NTRK2 | |
| - AGBL4-NTRK2 | |
| OLFM4 | Stomach Cancer |
| p53 | Sarcomas, Ovarian cancer |
| Parp+CDK12, EWS/FLI, | Sarcomas |
| PCDH10 | Glioma, Breast, Colorectal, Stomach, Kidney/Renal Cancer Liver cancer |
| PD-L1 | Stomach. Kidney/Renal. Liver Cancer |
| PD1 | Cervical Cancer |
| PDCD1 LG2 (also known as PD-L2), | Cervical Cancer |
| PDGF | Sarcomas |
| PDGFR | Glioma, Liver cancer |
| PDGFR(Alpha), PDGFR(Beta) | Liver cancer |
| PDL-1 | Breast, Colorectal, Lung Cancer |
| PHF10 | Stomach Cancer |
| PIP2 | Sarcomas |
| PIP3 | Sarcomas |
| PI3K | Sarcomas |
| PL3KCA | Cervical, Liver Cancer |
| PLAS3 | Stomach Cancer |
| PLK1 | Glioma |
| PLK3CA | Lung cancer |
| Prohibitin | Stomach Cancer |
| PTEN | Cervical, Lung, Stomach, Liver Cancer |
| PTPN3 | Glioma |
| PTPRZ1-MET | Glioma |
| PTTG1 | Glioma, Prostate Cancer |
| RAB40C | Stomach Cancer |
| RAF | Liver cancer |
| Raf | Sarcomas |
| RARB | Cervical Cancer |
| Ras | Stomach Cancer |
| RAS | Liver cancer, Sarcomas |
| RB | Sarcomas |
| RB1 | Glioma |
| RBL1 | Glioma |
| RECK | Stomach Cancer |
| RELA fusion | Glioma |
| RET | Lung cancer |
| RMBXL1 | Stomach Cancer |
| ROS-1 | Glioma Lung cancer |
| RPS6KA3 | Liver cancer |
| RRM2B | Liver cancer |
| RTK | Colorectal Cancer, Sarcomas |
| RUNX3 | Stomach Cancer |
| S-100A9 | Liver cancer |
| SHKBP1 | Cervical Cancer |
| SLC52A2 | Liver cancer |
| SMARCD1 | Stomach Cancer |
| SOX2 | Stomach Cancer |
| SPHK1 | Stomach Cancer |
| STAP-2 | Prostate Cancer |
| STARD13 | Glioma |
| STAT3 | Breast cancer |
| STMN1 | Stomach Cancer |
| STX6 | Liver cancer |
| TBX2 | Glioma |
| TERT | Liver cancer |
| TF | Prostate Cancer |
| TFP12 | Glioma |
| TGF-BetaR | Liver cancer |
| TGFBR2 | Cervical Cancer |
| THBS2 | Cervical Cancer |
| TIE2 | Liver cancer |
| TK1 | Prostate Cancer |
| TMEFF2 | Glioma |
| TMPRSS2-ERG | Prostate Cancer |
| TP53 | Liver cancer |
| TP53 | Lung cancer |
| UBE2C | Prostate Cancer |
| VEGF | Colorectal Cancer, Glioma, Sarcomas |
| VEGFA | Liver cancer |
| VEGFR | Kidney/Renal Cancer |
| VEGFR1-4 | Liver cancer |
| WDR79 | Breast Cancer |
| WDR79 | Colorectal Cancer |
| WDR79 | Lung cancer |
| YWAE/FAM22A/B | Sarcomas |
| ZEB1/ZEB2 | Stomach Cancer |
| ZFHX3 | Glioma |
| ZXH2 | Kidney/Renal cancer |

| **Table 8:** Other Ovarian Cancer Targets (some including target sequences) | | |
|---|---|---|
| **SEQ ID NO** | **Target** | **Detail/Targeting Sequence** |
| | 4EBP1 | shRNA against human 4E-BP1: (hshBP1)(sigma: TRCN0000040203): |
| 159. | | CCGGGCCAGGCCTTATGAAAGTGATCTCGAGATCACTTTCATAAGGCCTGGCTTTTTG |
| 160. | AKT | AKT1 TRCN0000010174 GGACTACCTGCACTCGGAGAA |
| 161. | | AKT2 TRCN0000000564 CTTCGACTATCTCAAACTCCT |
| 162. | | AKT3 TRCN0000010187 CTGCCTTGGACTATCTACATT |
| | ANG1/ANG2 and Tie, ATM, AXL, BCL-2, BET, bFGF, BRAF, BRIPI, CDC42, c-KIT CD40, CD184, CDCP1, CHK1, TNNB1, CX3CL1 aka Fractalkine, CX₃CR1 aka Fractalkine, CXCR4 | |
| 163. | DXL1 | The targeting sequence for Dlx1 was 5'-AACCGGAGGTTCCAACAAACT-3' |
| **164.** | | (sense strand: 5'-CCGGAGGTTCCAA-CAAACTTT-3'; |
| **165.** | | antisense strand 5'-AGTTTGTTGGAACCTCCGGTT-3') |
| | EGFR | |
| 166. | elF4E | shRNA1 - 5′- CACCGCCAAAGATAGTGATTGGTTATTTCAAGAGAATAACCAATCACTATCTTTGGTTT TTTG-3′; |
| 167. | | shRNA2 - 5′- CACCGGAGGACGATGGCTAATTACATTCAAGAGATGTAATTAGCCATCGTCCTCCTTTT TTG-3′; |
| 168. | | shRNA3 - 5′- CACCGTGGCGCTGTTGTTAATGTTATTCAAGAGATAACATTAACAACAGCGCCACTTTT TTG-3′ |
| | EMSY, ERBB1, Erb2, ETS1, EZH2, FAK, FAS, **FER,** FGF, FOS, FR-alpha, Flt3, FRA, GAB1, GADD45B, Grb2, HER2, HER3, HER4, ICOS, IDO, IGF, IGF-2, Insulin, IGF-1R, IGF-2R, IR, IL-6, JAK1, JAK2, JAK3, JAK4, KLF6, KRAS, MAPK | |
| 169. | March7 | shRNA 1 for MARCH7(NM_022826) AAGTGCTAGGATGATGTCTGGAA |
| 170. | | shRNA 2 for MARCH7(NM_022826) AAGAACAGATTCCTCTATTAGTA |
| 171. | | shRNA 3 for MARCH7(NM_022826) AAGATCTAGTCAGGATTCCTTGA |
| 172. | | shRNA 4 for MARCH7(NM_022826) AAGAGATGAATCTTCAAGGATAC |
| | MAST1, MDM2, MEK, MET, MMP9 | |
| 173. | mTOR | V2LHS_262100 Mature Antisense: TAGGAGGCAGCAGTAAATG; many others listed from company |
| | NOX1, OX40, PAK1, PARP, PDGF, PD-1, PDL1, PICT-1 aka GLTSCR2 | |
| 174. | PI3K | shRNA-1 (ccggccacttatgctttaccttctactcgagtagaaggtaaagcataagtggttttt) TRCN0000002228, |
| 175. | | shRNA-2 (ccgggctagtgtgaaggtctccattctcgagaatggagaccttcacactagcttttt) TRCN0000002229, |
| 176. | | shRNA-3 (ccggcaaagaagtatggaacgagtactc-gagtactcgttccatacttctttgttttt) TRCN0000002231, |
| 177. | | shRNA-4 (ccggcgagcagtagatcaagtaattctcgagaattacttgatctactgctcgttttt) TRCN0000002230 |
| | PIK3CA | |
| 178. | PRAS40 | The shRNA sequence of PRAS40 was GCTGAGTTCTAAGCTCTAA (sense) |
| | PRMT5, Raf, RAS, RB, Ror1, Ror2, Shp2, SLIT3, STAT, SYK, TGF alpha, TLR4, TNF-alpha, TNF, TOP1, TOP2, TP53, TRAIL, TYK2 | |
| 179. | USP7 | USP7 (GCGATTACAAGAAGAGAAA) through Dharmacon |
| 180. | USP15 | shRNA-1 5'-CCGGCCGTAATCAATGTGGGCCTATCTCGAGATAGGCCCACATTGATTACGGTTTTT-3'; |
| | VEGF, WDR77, WDR79, WEE1, XIAP, Y1349 | |

| **Table 9.** List of Cancer Gene Targets. | | |
|---|---|---|
| **Gene** | **Chromosome** | **Locus** |
| ABCB1 | 7 | 7q21.1 |
| ACVR1 B | 12 | 12q13 |
| AGTR1 | 3 | 3q21-q25 |
| AKT1 | 14 | 14q32.32 |
| AKT2 | 19 | 19q13.1-q13.2 |
| ALOX12 | 17 | 17p13.1 |
| ALOX5 | 10 | 10q11.2 |
| ALOX5AP | 13 | 13q12 |
| ANG | 14 | 14q11.1-q11.2 |
| ARHC | 1 | 1p21-p13 |
| ARMET | 3 | 3p21.1 |
| B3GALT5 | 21 | 21q22.3 |
| BAG3 | 10 | 10q25.2-q26.2 |
| BAG4 | 8 | 8p22 |
| BAX | 19 | 19q13.3-q13.4 |
| BHLHB2 | 3 | 3p26 |
| BIRC5 | 17 | 17q25 |
| BRCA2 | 13 | 13q12.3 |
| CCK | 3 | 3p22-p21.3 |
| CCKAR | 4 | 4p15.1-p15.2 |
| CCKBR | 11 | 11p15.4 |
| CCND3 | 6 | 6p21 |
| CD44 | 11 | 11p13 |
| CD9 | 12 | 12p13 |
| CDH1 | 16 | 16q22.1 |
| CDK4 | 12 | 12q14 |
| CDKN1A | 6 | 6p21.2 |
| CDKN2A | 9 | 9p21 |
| CDKN2B | 9 | 9p21 |
| CLDN4 | 7 | 7q11.23 |
| DAB2 | 5 | 5p13 |
| DCC | 18 | 18q21.3 |
| DUSP6 | 12 | 12q22-q23 |
| EGF | 4 | 4q25 |
| EGFR | 7 | 7p12 |
| EP300 | 22 | 22q13.2 |
| EPHB2 | 1 | 1p36.1-p35 |
| ERBB2 | 17 | 17q21.1 |
| EREG | 4 | 4 |
| FGFR1 | 8 | 8p11.2-p11.1 |
| FHIT | 3 | 3p14.2 |
| FRAP1 | 1 | 1p36.3-p36.2 |
| GAS | 17 | 17q21 |
| GLRX | 5 | 5q14 |
| GNG7 | 19 | 19p13.3 |
| GPI | 19 | 19q13.1 |
| GUSB | 7 | 7q21.11 |
| HK2 | 2 | 2p12 |
| HPSE | 4 | 4q21.3 |
| ID2 | 2 | 2p25 |
| IL8 | 4 | 4q13-q21 |
| IRS1 | 2 | 2q36 |
| IRS2 | 13 | 13q34 |
| KAI1 | 11 | 11p11.2 |
| KRAS2 | 12 | 12p12.1 |
| KRT20 | 17 | * |
| MADH4 | 18 | 18q21.1 |
| MADH6 | 15 | 15q21-q22 |
| MAP2K4 | 17 | 17p11.2 |
| MAP3K1 | 5 | 5 |
| MAPK14 | 6 | 6p21.3-p21.2 |
| MDM2 | 12 | 12q14.3-q15 |
| MADH7 | 18 | 18 |
| MEN1 | 11 | 11q13 |
| MET | 7 | 7q31 |
| MMP11 | 22 | 22q11.23 |
| MMP2 | 16 | 16q13-q21 |
| MMP3 | 11 | 11q22.3 |
| MMP7 | 11 | 11q21-q22 |
| MTA1 | * | * |
| MUC1 | 1 | 1q21 |
| MYC | 8 | 8q24.12-q24.13 |
| NFKB | ** | ** |
| NME1 | 17 | 17q21.3 |
| NTRK1 | 1 | 1q21-q22 |
| NTSR1 | 20 | 20q13-20q13 |
| P8 | 16 | * |
| PCD1 | 13 | 13q21.33 |
| PLAUR | 19 | 19q13 |
| PPARG | 3 | 3p25 |
| PRG1 | 19 | 19q13.2 |
| PRKCA | 17 | 17q22-q23.2 |
| PSCA | 8 | 8q24.2 |
| PTGS2 | 1 | 1q25.2-q25.3 |
| RABIF | 1 | 1q32-q41 |
| RAD51 | 15 | 15q15.1 |
| RB1 | 13 | 13q14.2 |
| RELA | 11 | 11q13 |
| RNASE1 | 14 | 14 |
| RPS6KB2 | 11 | 11cen-q12.1 |
| SLC16A7 | 12 | 12q13 |
| SCYB14 | 5 | 5q31 |
| SDC1 | 2 | 2p24.1 |
| SERPINB5 | 18 | 18q21.3 |
| SERPINI2 | 3 | 3q26.1-q26.2 |
| SLC2A1 | 1 | 1p35-p31.3 |
| SPINT2 | 19 | 19q13.1 |
| SST | 3 | 3q28 |
| STK11 | 19 | 19p13.3 |
| TDG | 12 | 12q24.1 |
| TEM7 | 17 | * |
| TFF1 | 21 | 21q22.3 |
| TFF2 | 21 | 21q22.3 |
| TGFA | 2 | 2p13 |
| TGFB1 | 19 | 19q13.1 |
| TGFBR1 | 9 | 9q22 |
| TGFBR2 | 3 | 3p22 |
| TIMP1 | x | xp11.3-p11.23 |
| TJP1 | 15 | 15q13 |
| TM4SF5 | 17 | 17p13.3 |
| TMPRSS4 | 11 | 11q23.3 |
| TNFRSF6 | 10 | 10q24.1 |
| TP53 | 17 | 17p13.1 |
| TSLC1 | * | * |
| TXN | 9 | 9q31 |
| UCHL1 | 4 | 4p14 |
| VEGF | 6 | 6p12 |
| VEGFC | 4 | 4q34.1-q34.3 |
| ZNF146 | 19 | 19q13.1 |

The following references are incorporated by reference in their entirety for all purposes.
ATCC Sarcoma Cell Lines. atcc.org/∼/media/PDFs/Cancer%20and%20Normal%20cell%20lines%20tables/Sarcoma%20cel 1%20lines.ashx
Catanese, D.J., et al., Supercoiled MiniVector DNA resists shear forces associated with gene therapy delivery, Gene Ther. 19(1): 94-100 (2012).
Chan D.W., et al., Overexpression of FOXM1 transcription factor is associated with cervical cancer progression and pathogenesis, J. Pathol. 215(3): 245-252 (2008).
Darquet A.M., et al., Minicircle: an improved DNA molecule for in vitro and in vivo gene transfer, Gene Ther., 6: 209-218 (1999).
Douard R., et al., Sonic Hedgehog-dependent proliferation in a series of patients with colorectal cancer, Surgery. 139(5): 665-670 (2006).
ES4: Cellosaurus, web.expasy.org/cellosaurus/CVCL_1200.
Fogg, J.M., et al., Exploring writhe in supercoiled minicircle DNA. J. Phys.-Condes. Matter, 18: S145-S159 (2006).
Hardee, C.L., Advances in Non-Viral DNA Vectors for Gene Therapy, Genes 8, 65 (2017)
Hornstein, B.D., et al., Effects of Circular DNA Length on Transfection Efficiency by Electroporation into HeLa Cells, PLoS One. 11(12): e0167537 (2016).
Jaiswal N., et al., Biology of FOXM1 and its emerging role in cancer therapy, J. Proteins Proteomics 5(1): 1-24 (2014).
Kalin T.V., et al., Increased levels of the FoxM1 transcription factor accelerate development and progression of prostate carcinomas in both TRAMP and LADY transgenic mice, Cancer Res. 66(3): 1712-1720 (2006).
Kalinichenko V.V., et al., Foxmlb transcription factor is essential for development of hepatocellular carcinomas and is negatively regulated by the pl9ARF tumor suppressor, Genes Dev., 18(7): 830-850 (2004).
Kannan K., CDKN2D- WDFY2 is a cancer-specific fusion gene recurrent in high-grade serous ovarian carcinoma, PLoS Genet. 10(3):e1004216 (2014).
Kannan K., et al., Recurrent BCAM-AKT2 fusion gene leads to a constitutively activated AKT2 fusion kinase in high-grade serous ovarian carcinoma, P.N.A.S. USA. 17;112(11):E1272-7 (2015).
Kelleher F.C. and O'sullivan H., FOXM1 in sarcoma: role in cell cycle, pluripotency genes and stem cell pathways, Oncotarget 7(27): 42792-42804 (2016).
Kim I.M., et al., The Forkhead Box ml transcription factor stimulates the proliferation of tumor cells during development of lung cancer, Cancer Res. 66(4): 2153-2161 (2006).
Kim J., et al., High-grade serous ovarian cancer arises from fallopian tube in a [Dicer-Pten double-knockout] mouse model, P.N.A.S. USA. 109(10):3921-6 (2012).
Liu M., et al., FoxM1B is overexpressed in human glioblastomas and critically regulates the tumorigenicity of glioma cells, Cancer Res. 66(7): 3593-3602 (2006).
Lokody, I., Signalling: FOXM1 and CENPF: co-pilots driving prostate cancer, Nature Reviews Cancer 14: 450-451 (2014).
Mitsiades, N., Fas-Mediated Apoptosis in Ewing's Sarcoma Cell Lines by Metalloproteinase Inhibitors, Journal of the National Cancer Institute, Volume 91, Issue 19: 1678-1684 (1999).
SK-ES-1: Human Ewing Sarcoma Cell Line, Memorial Sloan Kettering Cancer Center, https://www.mskcc.org/research-advantage/support/technology/tangible-material/sk-es-1-human-ewing-sarcoma-cell-line
Teh M.T., et al., FOXM1 is a downstream target of Gli1 in basal cell carcinomas, Cancer Res., 62(16): 4773-4780 (2002).
US20150376645, US20140056868, 61/653,279, filed May 30, 2012, Supercoiled MiniVectors as a tool for DNA repair, alteration and replacement
Wang Z., et al., Down-regulation of Forkhead Box M1 transcription factor leads to the inhibition of invasion and angiogenesis of pancreatic cancer cells, Cancer Res. 67(17): 8293-8300 (2007).
WE68: Cellosaurus, web.expasy.org/cellosaurus/CVCL_9717.
Wei, Ping et al., FOXM1 promotes lung adenocarcinoma invasion and metastisis by upregulating snail, International Journal of Biological Sciences 11(2): 186-198 (2015).
Wonsey D.R., et al., Loss of the forkhead transcription factor FoxM1 causes centrosome amplification and mitotic catastrophe, Cancer Res., 65(12): 5181-5189 (2005).
Zhang X, et al., Knockdown of CIP2A sensitizes ovarian cancer cells to cisplatin: an in vitro study, Int. J. Clin. Exp. Med. 8(9):16941-16947 (2015).
Zhang X. et al., A novel FOXM1 isoform, FOXM1D, promotes epithelial-mesenchymal transition and metastasis through ROCKs activation in colorectal cancer, Oncogene 36: 807-819 (2017).
Zhao N., et al., Transfection of shRNA-encoding MiniVector DNA of a few hundred base pairs to regulate gene expression in lymphoma cells, Gene Ther. 18(3):220-4 (2011).
Zona S., et al., FOXM1: An emerging master regulator of DNA damage response and genotoxic agent resistance, Biochim Biophys Acta., 1839(11): 1316-1322 (2014).
US8460924, US8729044, US9267150, US20110160284, US20120302625, US20130316449, 61/252,455, filed Oct. 16, 2009, Supercoiled MiniVectors for gene therapy applications
US7622252, US20070020659, 60/689,298, filed Jun. 10, 2005, Generation of minicircle DNA with physiological supercoiling
US20060211117 Methods of making minicircles
WO1994009127 Supercoiled minicircle DNA as a unitary promoter vector
WO2002083889 Methods for the production of minicircles
62/470,997, Mar 14, 2017, TARGETING MINIVECTORS TO SPECIFIC TISSUE USING SHAPE
IMPROVING NON-VIRAL GENE DELIVERY: POLYMER CARRIERS FOR SPATIAL AND TEMPORAL CONTROL OF NUCLEIC ACID RELEASE, Abbygail A. A. Foster, University of Delaware Chemical Engineering Ph.D. thesis dissertation, Spring 2014
Ramamoorth M., & Narvekar, A., Non Viral Vectors in Gene Therapy- An Overview, J. Clinical & Diagnostic Res. 2015 Jan, Vol-9(1): GE01-GE06.
Hidai C., & Kitano, H., Nonviral Gene Therapy for Cancer: A Review, Diseases 2018,6,57.
Lijun Qian et al, The present and future role of ultrasound targeted microbubble destruction in preclinical studies of cardiac gene therapy, J. Thoracic Dis., 2018;10(2):1099-1111.

## Claims

1. A MiniVector, said MiniVector being a double stranded, supercoiled, and circular DNA encoding an ovarian cancer inhibitory sequence (OCi) that can be expressed in a mammalian cell, said MiniVector lacking a bacterial origin of replication and lacking an antibiotic resistance gene.

2. The MiniVector of claim 1, wherein said MiniVector is at least 97% pure.

3. The MiniVector of claim 1, wherein said MiniVector is at least 97% pure, and is separated from a parent plasmid and recombination side-products on the basis of size, and does not use a restriction enzyme cleavage *in vivo* for preparation of said MiniVector.

4. The MiniVector of claim 1, wherein said OCi encodes an inhibitory RNA for a target gene selected from shRNA, miRNA, lncRNA, piRNA, RNAi, or antisense RNA.

5. The MiniVector of claim 1, wherein said OCi encodes an inhibitory RNA for a target gene selected from FOXM1, AKT, CENPA, PLK1, CDC20, BIRC5, AURKB, CCNB1, CDKN3, BCAM-AKT2, CDKN2D-WDFY2, SLC25A6, CIP2A, CD133, ALDH1A1, CD44, SALL4, and/or PRDM16, alone, or in combination, and wherein expression of said target gene is reduced at least 10% by said inhibitory RNA when said MiniVector is introduced into mammalian cells and expressed therein.

6. The MiniVector of claim 1, wherein said ovarian cancer inhibitory sequence is an apoptosis gene selected from p53, p16, p21, p27, E2F genes, FHIT, PTEN, and/or CASPASE alone, or in combination, and said apoptosis gene is overexpressed when said MiniVector is introduced into mammalian cells.

7. The MiniVector of claim 1, comprising a promoter operably connected to said OCi operably connected to a terminator.

8. The MiniVector of claim 1, comprising a promoter connected to said OCi operably connected to a terminator, and additionally comprising an enhancer sequence and/or a nuclear localization signal.

9. The MiniVector of claim 1, that is made by:
a) engineering a parent plasmid DNA molecule comprising site-specific recombination sites on either side of said OCi;
b) transforming said parent plasmid into a cell suitable for site-specific recombination to occur, under conditions such that topoisomerase IV decatenation activity is inhibited, thereby producing a plurality of catenated DNA circles, wherein at least one of the circles in each catenane is a supercoiled DNA MiniVector of less than about 5 kb in length;
c) decatenating the catenated site-specific recombination products, thereby releasing the supercoiled DNA MiniVector from the catenanes; and
d) isolating the supercoiled DNA MiniVector.

10. The MiniVector of claim 1, wherein said MiniVector is ≤600 bp in length, excluding said OCi.

11. A composition comprising a MiniVector in a pharmaceutically acceptable excipient, said MiniVector being a double-stranded, supercoiled, nicked, or relaxed circular DNA encoding an OCi and lacking a bacterial origin of replication and lacking an antibiotic resistance gene, wherein said circular DNA is at least 95% free of parent plasmid DNA or recombination side-products, wherein said OCi is expressible in human cells and thereby inhibits the expression of a human target gene selected from FOXM1, AKT, CENPA, PLK1, CDC20, BIRC5, AURKB, CCNB1, CDKN3, BCAM-AKT2, CDKN2D-WDFY2, SLC25A6, CIP2A, CD133, ALDH1A1, CD44, SALL4, and/or PRDM16, alone, or in any combination.

12. The composition of claim 11, wherein said MiniVectors are 250 bp to 5,000 bp in total length; or
wherein said MiniVector is ≤600 bp in length, excluding said OCi; or
wherein said MiniVector is ≤250 bp in length, excluding said OCi; or
wherein said ovarian cancer inhibitory sequence is codon optimized for humans or human cancers; or
wherein said MiniVector is CpG-free, CpG maximized, or CpG minimized; or
wherein said MiniVector is supercoiled; or
wherein said MiniVector has a specific DNA sequence-defined shape.

13. A MiniVector, said MiniVector being a double-stranded, supercoiled, circular DNA encoding an OCi that can be expressed in a mammalian cell, wherein said OCi encodes an inhibitory RNA for a target gene selected from FOXM1, AKT, CENPA, PLK1, CDC20, BIRC5, AURKB, CCNB1, CDKN3, BCAM-AKT2, CDKN2D-WDFY2, SLC25A6, CIP2A, CD133, ALDH1A1, CD44, SALL4, and/or PRDM16, alone or in any combination, wherein said MiniVector lacks a bacterial origin of replication and lacks an antibiotic resistance gene, and wherein said MiniVector is made by:
a) engineering a parent plasmid DNA molecule comprising site-specific recombination sites on either side of said OCi;
b) transforming said parent plasmid into a cell suitable for site-specific recombination to occur, under conditions such that topoisomerase IV decatenation activity is inhibited, thereby producing a plurality of catenated DNA circles, wherein at least one of the circles in each catenane is a supercoiled DNA MiniVector of less than about 5 kb in length;
c) decatenating the catenated site-specific recombination products, thereby releasing the supercoiled DNA MiniVector from the catenanes; and
d) isolating the supercoiled DNA MiniVector.

14. The MiniVector of claim 13, or cells containing same, for use in treating ovarian cancer, wherein said OCi inhibits expression of said target gene in said patient by at least 20%.
